(19) Europäisches Patentamt / European Patent Office / Office européen des brevets

(11) **EP 2 826 821 A1**

(12) **EUROPEAN PATENT APPLICATION**
published in accordance with Art. 153(4) EPC

(43) Date of publication:
**21.01.2015 Bulletin 2015/04**

(21) Application number: **13760756.0**

(22) Date of filing: **12.03.2013**

(51) Int Cl.:
***C09B 57/10*** *(2006.01)* ***H01L 31/04*** *(2014.01)*
***H01M 14/00*** *(2006.01)*

(86) International application number:
**PCT/JP2013/056724**

(87) International publication number:
**WO 2013/137221 (19.09.2013 Gazette 2013/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.03.2012 JP 2012061232**

(71) Applicant: **Fujifilm Corporation**
**Minato-ku**
**Tokyo 106-8620 (JP)**

(72) Inventors:
- **SASAKI, Kouitsu**
  **Ashigarakami-gun**
  **Kanagawa 258-8577 (JP)**
- **NOMURA, Kimiatsu**
  **Ashigarakami-gun**
  **Kanagawa 258-8577 (JP)**
- **KOBAYASHI, Katsumi**
  **Ashigarakami-gun**
  **Kanagawa 258-8577 (JP)**

(74) Representative: **Hoffmann Eitle**
**Patent- und Rechtsanwälte PartmbB**
**Arabellastraße 30**
**81925 München (DE)**

(54) **METAL COMPLEX DYE, PHOTOELECTRIC CONVERSION ELEMENT, DYE-SENSITIZED SOLAR CELL, DYE ADSORPTION LIQUID COMPOSITION FOR DYE-SENSITIZED SOLAR CELL, SEMICONDUCTOR ELECTRODE FOR DYE-SENSITIZED SOLAR CELL, AND METHOD FOR PRODUCING DYE-SENSITIZED SOLAR CELL**

(57) A metal complex dye represented by the following formula (I), photoelectric conversion element, and dye-sensitized solar cell.

$M(LD)(LA)\cdot(Y)_{mY}$ formula (I)

wherein, in formula (I), M represents $Fe^{2+}$, $Ru^{2+}$ or $Os^{2+}$;
LD represents a tridentate ligand represented by the following formula (A);
LA represents a specific tridentate ligand wherein three cyclic coordinating groups different from LA are linked;

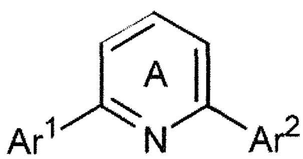

Formula (A)

wherein, in formula (A), $Ar^1$ and $Ar^2$ each independently represent specific heterocyclic groups such as a benzene ring group or a triazole ring group or the like. Y represents a counter ion required for neutralizing an electric charge, and mY represents an integer of 0 to 2.

EP 2 826 821 A1

Printed by Jouve, 75001 PARIS (FR)

**(Cont. next page)**

*Fig. 1*

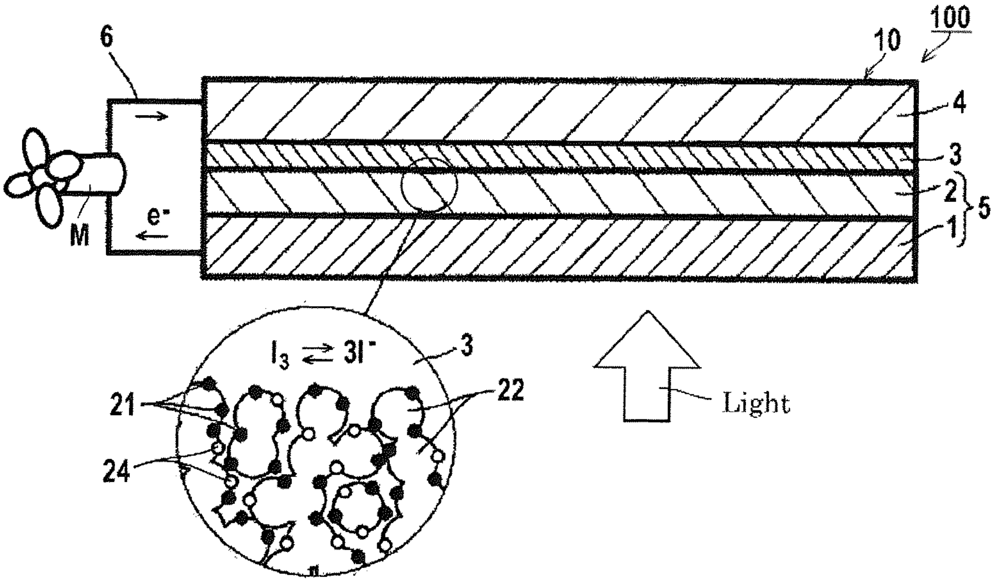

## Description

### TECHNICAL FIELD

The present invention relates to a metal complex dye, a photoelectric conversion element, a dye-sensitized solar cell, a dye adsorption liquid composition for the dye-sensitized solar cell, a semiconductor electrode for the dye-sensitized solar cell, and a method for producing the dye-sensitized solar cell.

### BACKGROUND ART

Photoelectric conversion elements are used in various photosensors, copying machines, solar cells or the like, and various systems have been studied and put in practical use. Specific examples include elements utilizing, as a photoelectric conversion material therefor, a metal, a semiconductor, an organic pigment or a dye. A background includes expectation of the solar cell that makes use of non-exhaustive solar energy as one utilizing inexhaustible clean energy without needing a fossil fuel. Above all, research and development of silicon-based solar cells have long been in progress. Based on a policy-wise consideration in each country, widespread use thereof is still in progress. However, silicon is an inorganic material, and has its own limitations in terms of throughput and molecular modification.

Under such circumstances, research is being vigorously carried out on dye-sensitized solar cells. Especially, to have built momentum toward such research is research results by Graetzel et al. of École Polytechnique Fédérale de Lausanne in Switzerland. They employed a structure in which a dye formed from a ruthenium complex was fixed at the surface of a porous titanium oxide thin film, and realized a conversion efficiency that was comparable to that of amorphous silicon. Thus, the dye-sensitized solar cells instantly attracted the attention of researchers all over the world.

Development of a ruthenium complex-based sensitizing dye is continued by applying this technology toward improvement in photoelectric conversion efficiency. Specific examples include a metal complex commonly known as N3, N749 (Black Dye), N719 (Red Dye), PRT4, TF-3 or TF-4 (for example, see Patent Literature 1, 2, Non-Patent Literature 1).

More recently in particular, solar sells get attention to and raise expectation for energy sources in place of nuclear power generation, and more improvement in performance of the solar sell has been required.

### CITATION LIST

#### Patent Literatures

Patent Literature 1: US Patent Application Publication No. 2010/0258175A1
Patent Literature 2: Japanese Patent No. 4298799

#### Non-Patent Literature

Non-Patent Literature 1: Angew. Chem. Int. Ed., 2011, 50, 2054-2058

### SUMMARY OF THE INVENTION

### TECHNICAL PROBLEM

A performance level required for a solar cell is increasing year after year, but durability is not necessarily satisfied, and satisfaction of both of improvement in short-circuit current density (Jsc) and improvement in the durability is particularly desired. Furthermore, upon satisfaction of both thereof, adsorption properties of a metal complex dye onto semiconductor fine particles (strong adsorption power and a high adsorption rate) are required, and solution stability of the metal complex dye is not necessarily satisfied, and thus further improvement has been required. In addition thereto, a deviation of performance between preparation lots of prepared photoelectric conversion elements has been observed. In view of such a current situation in the present technical field, an object of the present invention is to provide a metal complex dye, a photoelectric conversion element, a dye-sensitized solar cell, a dye adsorption liquid composition for the dye-sensitized solar cell, a semiconductor electrode for the dye-sensitized solar cell, and a method for producing the dye-sensitized solar cell, in which performance excellent in cell characteristics as a photoelectric conversion element is exhibited, such as high photoelectric conversion efficiency ($\eta$), and also both of improvement in short-circuit current density (Jsc), and improvement in durability are satisfied, adsorption properties are improved, solution stability of the

metal complex dye is improved, and a lot-to-lot difference in performance of the photoelectric conversion elements is reduced.

SOLUTION TO PROBLEM

The present inventors studied durability in various manners, and as a result, found that a conventional ruthenium metal complex such as Black Dye or Red Dye has a thiocyanate ion ($[NCS]^-$) as a donor ligand, and therefore such a ligand receives a nucleophilic attack by water or the like, and durability is decreased. Therefore, the present inventors studied metal complex dyes having no such ligands in various manners, and consequently found that a conventional metal complex dye having both donor and acceptor ligands as a tridentate ligand has a problem of shifting a long wave end of absorption in a light absorption spectrum to a short wave, resulting in a decrease in short-circuit current density (Jsc).

On the other hand, the present inventors adjusted, as a ligand on a donor side, HOMO (highest occupied molecular orbital) and LUMO (lowest unoccupied molecular orbital) of an electron donating metal complex dye to synthesize and search various metal complex dyes. For example, the present inventors studied an increase in a transition dipole moment in a direction from a donor ligand side toward an acceptor ligand and an increase in the transition dipole moment in a direction toward a left-right coordination parts connecting with a tridentate central coordination part perpendicular thereto, and found that the short-circuit current density (Jsc) is improved to allow satisfaction of both of the density and the durability. Eventually, the present inventors arrived at the present invention by structure search of various kinds of acceptor side ligands and further study on a combination thereof.

That is, the tasks of the present invention can be achieved by the following means.

(1) A metal complex dye represented by the following formula (I):

$M(LD)(LA)\cdot(Y)_{mY}$ formula (I)

wherein, in formula (I), M represents $Fe^{2+}$, $Ru^{2+}$ or $Os^{2+}$;
LD represents a tridentate ligand represented by the following formula (A);
LA represents a tridentate ligand represented by the following formula (B);
Y represents a counter ion required for neutralizing an electric charge; and
mY represents an integer of 0 to 2,

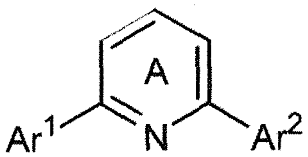

Formula (A)

wherein, in formula (A), $Ar^1$ and $Ar^2$ each independently represent a group selected from a benzene ring group, a pyrrole ring group, a pyrazole ring group, an imidazole ring group, a pyrazine ring group, a pyrimidine ring group, a pyridazine ring group, a triazole ring group, a triazine ring group, a tetrazole ring group, a quinoline ring group, an isoquinoline ring group, a quinazoline ring group, a thiazole ring group, an isothiazole ring group, an oxazole ring group, an isoxazol ring group, a furan ring group, a thiophene ring group, a pyrrolidine ring group, a piperidine ring group, a morpholine ring group, a piperazine ring group, a tetrahydrofuran ring group, a tetrahydropyran ring group, a 4H-pyran ring group, a 1,4-dihydropyridine ring group, a tetradehydromorpholine ring group, a tetradehydrothiomorpholine ring group and a group in which a benzene ring is condensed to the cyclic groups; provided that, when one of $Ar^1$ and $Ar^2$ is a pyrazole ring group, the other is selected from the above-described cyclic groups other than the pyrazole ring group; the cyclic group of $Ar^1$ and $Ar^2$ may be an anion in which a hydrogen atom is deprotonated; all of $Ar^1$, $Ar^2$ and a pyridine ring A may have a substituent other than a carboxyl group, and in a case where two or more substituents exist, these may be bonded to form a ring; $Ar^1$ and $Ar^2$ coordinate with M via a nitrogen atom having a lone electron pair, a nitrogen atom having an anion or a carbon atom having an anion, and

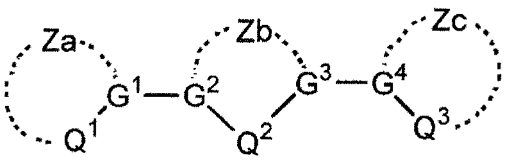

Formula (B)

wherein, in formula (B), Za, Zb and Zc each independently represent a group of atoms for forming a 5- or 6-membered

ring; in which at least one of the rings formed by Za, Zb and Zc has an acidic group;
$Q^1$ to $Q^3$ each independently represent a nitrogen atom having a lone electron pair, a nitrogen atom having an anion, or a carbon atom having an anion;
$G^1$ to $G^4$ each independently represent a carbon atom or a nitrogen atom; and
each bond of $G^1$-$Q^1$, $G^2$-$Q^2$, $G^3$-$Q^2$ and $G^4$-$Q^3$ represents a single bond or a double bond.

(2) The metal complex dye described in (1), wherein LD is represented by the following formula (A1):

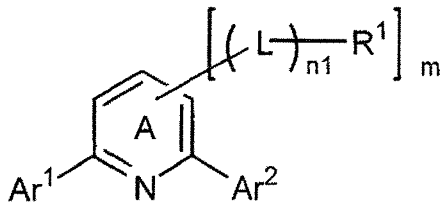

Formula (A1)

wherein, in formula (A1), $Ar^1$ and $Ar^2$ have the same meanings as those in formula (A);
L represents a divalent linking group; n1 represents an integer of 0 to 3;
$R^1$ represents an alkyl group, a cycloalkyl group, an alkenyl group, a cycloalkenyl group, an alkynyl group, an aryl group, a heterocyclic group, an alkoxy group, a cycloalkyloxy group, a cycloalkenyloxy group, an alkynyloxy group, an aryloxy group, a heterocyclic oxy group, an alkylthio group, a cycloalkylthio group, a cycloalkenylthio group, an alkynylthio group, an arylthio group, a heterocyclic thio group, an amino group, an alkylamino group, a cycloalkylamino group, an alkenylamino group, a cycloalkenylamino group, an arylamino group, a heterocyclic amino group, a silyl group, a silyloxy group or a halogen atom; and
m represents an integer of 0 to 3.

(3) The metal complex dye described in (2), wherein $R^1$ is represented by the following formula (A1-1) or (A1-2):

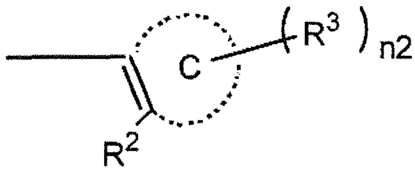

Formula (A1-1)

wherein, in formula (A1-1), ring C represents an aromatic ring or a heteroaromatic ring; $R^2$ and $R^3$ each independently represent a substituent; provided that the formula (A1-1) does not represent the following formula (A1-2);
n2 represents an integer of 0 to 4; When n2 is one or more, $R^2$ and $R^3$ may be bonded to form a ring; and when n2 is two or more, plural $R^3$'s may be bonded with each other to form a ring,

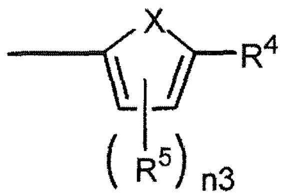

Formula (A1-2)

wherein, in formula (A1-2), X represents -O-, -N(Rz)-, -S- or -Se-;
$R^4$ represents a hydrogen atom, an alkyl group, an alkenyl group, an alkynyl group, an alkoxy group, an alkylthio group, an aryl group, an aryloxy group, an arylthio group, a silyloxy group, an amino group, a silyl group, a heterocyclic group or a halogen atom; Rz represents an alkyl group, an alkenyl group, an alkynyl group, an aryl group, a silyl group or a heterocyclic group;
$R^5$ represents an alkyl group, an alkenyl group, an alkynyl group, an alkoxy group, an alkylthio group, an aryl group, an aryloxy group, an arylthio group, a silyloxy group, an amino group, a silyl group, a heterocyclic group or a halogen atom;
n3 represents an integer of 0 to 2; when n3 is one or more, $R^4$ and $R^5$ may be bonded to form a ring; and when n3 is 2, and plural $R^5$'s may be bonded with each other to form a ring.

(4) The metal complex dye described in any one of (1) to (3),
wherein a coordinating atom of one of $Ar^1$ and $Ar^2$ is a carbon atom.

(5) The metal complex dye described in any one of (1) to (4),

wherein LA is represented by any one of the following formulas (B1) to (B8),

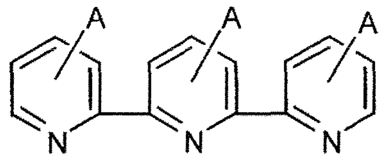

Formula (B1)

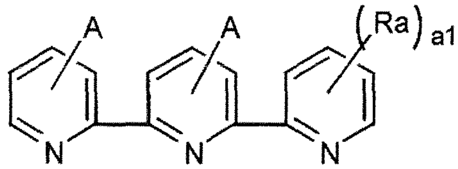

Formula (B2)

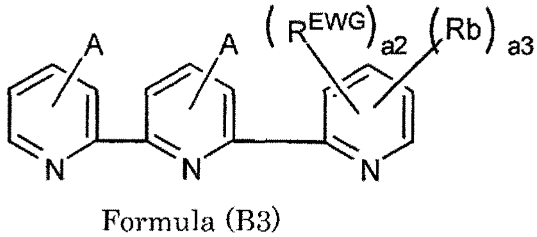

Formula (B3)

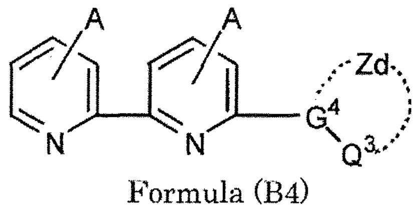

Formula (B4)

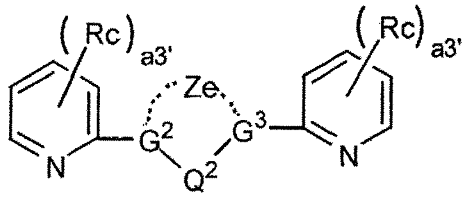

Formula (B5)

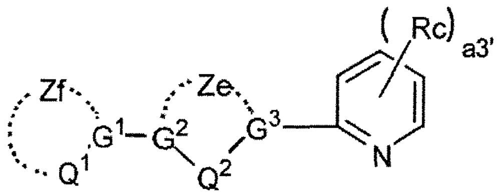

Formula (B6)

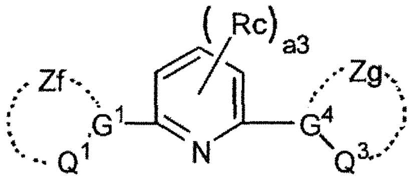

Formula (B7)

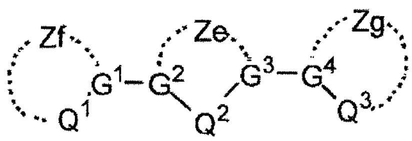

Formula (B8)

wherein $Q^1$ to $Q^3$ and $G^1$ to $G^4$ have the same meanings as those in formula (B), respectively;
Zd represent a group of atom for forming a benzene ring, a naphthalene ring, an anthracene ring, a phenanthrene ring, a pyrene ring, a perylene ring, a pyrrole ring, an indole ring, an imidazole ring, a benzoimidazole ring, a pyrazole ring, a pyrazine ring, a pyrimidine ring, a pyridazine ring, a triazole ring, a benzotriazole ring, a tetrazole ring, an indazole ring, a triazine ring, a purine ring, a quinoline ring, an isoquinoline ring, a quinazoline ring, a thiazole ring, a benzothiazole ring, an isothiazole ring, an oxazole ring, an isoxazole ring, a furan ring, a benzo[b]furan ring, a thiophene ring, a benzo[b]thiophene ring, a pyrrolidine ring, a piperidine ring, a morpholine ring, a piperazine ring, a tetrahydrofuran ring, a tetrahydropyran ring, a 4H-pyran ring, a 1,4-dihydropyridine ring, a tetradehydromorpholine ring, or a tetradehydrothiomorpholine ring;
Ze to Zg represent a group of atom for forming a 5- or 6-membered ring other than a pyridine ring;
A represents an acidic group;
Ra represents an aryl group, a heteroaryl group, an alkyl group, a cycloalkyl group, an alkoxy group, or a cycloalkoxy group; in which the alkyl group has a tertiary or quaternary carbon atom, and the alkoxy group has a tertiary or quaternary carbon atom, or a carbon atom directly binding to an oxygen atom of the alkoxy group is a secondary or tertiary carbon atom; a1 represents an integer of 1 or 2;
$R^{EWG}$ represents an electron-withdrawing group;
Rb and Rc represent a substituent;
a2 represents an integer of 1 to 4; a3 represents an integer of 0 to 3; a3' represents an integer of 0 to 4; in which the sum of a2 and a3 is an integer of 1 to 4; and

the ligands represented by any one of formulas (B5) to (B8) each have at least one acidic group.

(6) The metal complex dye described in any one of (1) to (5),
wherein Y in formula (I) is a halogen ion, an arylsulfonate ion, an aryldisulfonate ion, an alkylsulfate ion, a sulfate ion, a thiocyanate ion, a perchlorate ion, a tetrafluoroborate ion, a hexafluorophosphate ion, an acetate ion, a trifluoromethanesulfonate ion, an ammonium ion, an alkali metal ion or a hydrogen ion.

(7) A photoelectric conversion element, containing:

at least a photoconductor layer; and
an electrolyte,
wherein a semiconductor and the metal complex dye described in any one of (1) to (6) are contained in the photoconductor layer.

(8) The photoelectric conversion element described in (7),
wherein a redox-based compound contained in the electrolyte is a cobalt complex.

(9) A dye-sensitized solar cell, consisting of the photoelectric conversion element described in (7) or (8).

(10) A dye adsorption liquid composition for a dye-sensitized solar cell, consisting of 0.001 to 0.1 mass% of the metal complex dye described in any one of (1) to (6) in an organic solvent, provided that the dye adsorption composition for a dye-sensitized solar cell contains 0.1 mass% or less of water.

(11) A semiconductor electrode for a dye-sensitized solar cell, wherein a metal complex dye is carried on a surface of a semiconductor of a semiconductor electrode by using the liquid composition described in (10).

(12) A method of producing a dye-sensitized solar cell, comprising the step of:

making a surface of a semiconductor of a semiconductor electrode carry a metal complex dye by using the liquid composition described in (10).

In the present specification, unless otherwise specified, with respect to the carbon-carbon double bond, in a case where the E configuration or the Z configuration exists in the molecule, it may be either one of the two configurations or a mixture thereof. When there are two or more substituents, linking groups, ligands or the like (hereinafter referred to as substituents or the like) represented by a specific symbol, or when two or more substituents or the like are defined at the same time or alternatively, each of the substituents or the like may be the same or different from one another, unless otherwise specified. This is also applied to definition of the number of substituents or the like. Further, when a plurality of substituents or the like are close to one another (particularly adjacent to each other), they may be linked to one another to form a ring, unless otherwise specified. Further, a ring, for example, an aliphatic ring, an aromatic ring, or a heterocycle, may be ring-fused to form a fused ring.

In the present specification, each substituent may be further substituted with another substituent, unless otherwise specified.

## ADVANTAGEOUS EFFECTS OF INVENTION

The present invention can provide a metal complex dye, a photoelectric conversion element, a dye-sensitized solar cell, a dye adsorption liquid composition for the dye-sensitized solar cell, a semiconductor electrode for the dye-sensitized solar cell, and a method for producing the dye-sensitized solar cell, in which performance excellent in cell characteristics as a photoelectric conversion element is exhibited, such as high photoelectric conversion efficiency, and also both of improvement in short-circuit current density (Jsc), and improvement in durability are satisfied, adsorption properties are improved, solution stability of the metal complex dye is improved, and a lot-to-lot difference in performance of the photoelectric conversion elements is reduced.

Other and further features and advantages of the invention will appear more fully from the following description, appropriately referring to the accompanying drawings.

## BRIEF DESCRIPTION OF THE DRAWING

{Fig. 1} Fig. 1 is a cross-sectional view schematically showing one embodiment of the photoelectric conversion element of the present invention, including an enlarged view of a circular portion in the layer thereof.
{Fig. 2} Fig. 2 is a cross-sectional view schematically showing the dye-sensitized solar cell, prepared in Example 1.
{Fig. 3} Fig. 3 is a cross-sectional view schematically showing a modification example of the photoelectric conversion element as shown in Fig. 1 in its enlarged portion (circle), with respect to the dye-sensitized solar cell prepared in

Example 2 in which a cobalt complex was used in an electrolyte.

MODE FOR CARRYING OUT THE INVENTION

The present invention will be described in detail below.

First, a metal complex dye according to the present invention will be described in detail.

« Metal complex dye »

A metal complex dye according to the present invention is represented by the following formula (I).

$M(LD)(LA)\cdot(Y)_{mY}$ formula (I)

wherein, in formula (I), M represents $Fe^{2+}$, $Ru^{2+}$ or $Os^{2+}$;
LD represents a tridentate ligand represented by the following formula (A);
LA represents a tridentate ligand represented by the following formula (B);
Y represents a counter ion required for neutralizing an electric charge, and mY represents an integer of 0 to 2;

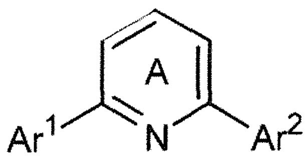

Formula (A)

wherein, in formula (A), $Ar^1$ and $Ar^2$ each independently represent a group selected from a benzene ring group, a pyrrole ring group, a pyrazole ring group, an imidazole ring group, a pyrazine ring group, a pyrimidine ring group, a pyridazine ring group, a triazole ring group, a triazine ring group, a tetrazole ring group, a quinoline ring group, an isoquinoline ring group, a quinazoline ring group, a thiazole ring group, an isothiazole ring group, an oxazole ring group, an isoxazol ring group, a furan ring group, a thiophene ring group, a pyrrolidine ring group, a piperidine ring group, a morpholine ring group, a piperazine ring group, a tetrahydrofuran ring group, a tetrahydropyran ring group, a 4H-pyran ring group, a 1,4-dihydropyridine ring group, a tetradehydromorpholine ring group, a tetradehydrothiomorpholine ring group and a group in which a benzene ring is condensed to the cyclic groups; however, when one of $Ar^1$ and $Ar^2$ is a pyrazole ring group, the other is selected from the above-described cyclic group other than the pyrazole ring group; the cyclic group of $Ar^1$ and $Ar^2$ may be an anion in which a hydrogen atom is deprotonated; all of $Ar^1$, $Ar^2$ and a pyridine ring A may have a substituent other than a carboxyl group, and when two or more substituents exist, these may be bonded to form a ring; $Ar^1$ and $Ar^2$ are a nitrogen atom having a lone electron pair, a nitrogen atom having an anion or a carbon atom having an anion, and coordinated to M;

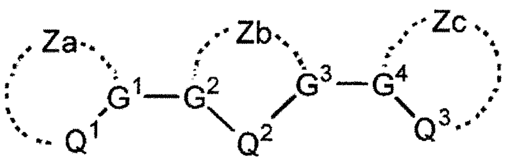

Formula (B)

wherein, in formula (B), Za, Zb and Zc each independently represent a group of atoms for forming a 5- or 6-membered ring; in which at least one of the rings formed by Za, Zb and Zc has an acidic group;
$Q^1$ to $Q^3$ each independently represent a nitrogen atom having a lone electron pair, a nitrogen atom having an anion, or a carbon atom having an anion; and
$G^1$ to $G^4$ each independently represent a carbon atom or a nitrogen atom.

Here, a bond of $G^1$-$Q^1$, $G^2$-$Q^2$, $G^3$-$Q^2$ or $G^4$-$Q^3$ represents either a single bond or a double bond.

<Ligand LD>

In the present invention, the ligand LD is categorized as a donor ligand, and is represented by formula (A).

A pyridine ring A may have a substituent other than a carboxyl group, and includes, preferably, a substituent other than the below-mentioned acidic group. Specific examples of such substituents include a substituent T other than the below-mentioned carboxyl group, and preferably, other than the acidic group, and includes, more preferably, an alkyl

group (preferably, an unsubstituted alkyl group, a halogenated alkyl group), a cycloalkyl group, an alkenyl group, a cycloalkenyl group, an alkynyl group, an aryl group, a heterocyclic group, an alkoxy group, a cycloalkyloxy group, a cycloalkenyloxy group, an alkynyloxy group, an aryloxy group, a heterocyclic oxy group, an alkylthio group, a cycloalkylthio group, a cycloalkenylthio group, an alkynylthio group, an arylthio group, a heterocyclic thio group, an amino group, an alkylamino group, a cycloalkylamino group, a cycloalkenylamino group, an arylamino group, a heterocyclic amino group, a silyl group, a silyloxy group or a halogen atom. The further preferred substituent herein is referred to as a substituent FT.

Among the substituents, an alkylene group, an alkynylene group, an arylene group, a hetero arylene group, and a group in which these groups are combined (however, a terminal is bonded with a hydrogen atom or a substituent to form a monovalent group) are further preferred, and a group represented by the following formula (Aa) is still further preferred.

$$-\left(L\right)_{n1}-R^1 \qquad \text{Formula (Aa)}$$

In the formula, L represents a divalent linking group. n1 represents an integer of 0 to 3.

$R^1$ represents an alkyl group, a cycloalkyl group, an alkenyl group, a cycloalkenyl group, an alkynyl group, an aryl group, a heterocyclic group, an alkoxy group, a cycloalkyloxy group, a cycloalkenyloxy group, an alkynyloxy group, an aryloxy group, a heterocyclic oxy group, an alkylthio group, a cycloalkylthio group, a cycloalkenylthio group, an alkynylthio group, an arylthio group, a heterocyclic thio group, an amino group, an alkylamino group, a cycloalkylamino group, an alkenylamino group, a cycloalkenylamino group, an arylamino group, a heterocyclic amino group, a silyl group, a silyloxy group or a halogen atom.

L may have a substituent other than a carboxyl group, and includes, preferably, a substituent other than the below-mentioned acidic group. Specific examples of such substituents include a substituent T other than the below-mentioned carboxyl group, and preferably, other than the acidic group, and more preferably, include the above-described substituent FT.

Specific examples of the divalent linking group of L include an alkylene group, an alkenylene group, an alkynylene group, an arylene group and a heteroarylene group, preferably, an alkenylene group, an alkynylene group, an arylene group and a heteroarylene group, and more preferably, an alkenylene group and an alkynylene group.

The alkenylene group in L preferably includes an ethynylene group, and the alkynylene group preferably includes an ethenylene group.

When n1 is an integer of 1 to 3, -{L)n1- includes, preferably, a group in which one to three ethynylene groups are linked (preferably, n1 is 1 or 2, and more preferably, 1), a group in which one to three ethenylene groups are linked (preferably, n1 is 1 or 2, and more preferably, 1) and a group in which an ethynylene group and an ethenylene group are combined, further preferably, one ethynylene group (n1 is 1) or one ethenylene group (n1 is 1), and still further preferably, one ethenylene group (n1 is 1).

The pyridine ring A may have the group represented by the formula (Aa), and also a substituent other than the group, and the group includes a substituent other than a carboxyl group, and preferably, a group other than the below-mentioned acidic group. Specific examples of such substituents include a substituent T other than the below-mentioned carboxyl group, and preferably, other than the acidic group, and include more preferably, the above-described substituent FT.

n1 represents an integer of 0 to 3, preferably, 0 to 2, and more preferably, 0 or 1.

$R^1$ represents an alkyl group, a cycloalkyl group, an alkenyl group, a cycloalkenyl group, an alkynyl group, an aryl group, a heterocyclic group, an alkoxy group, a cycloalkyloxy group, a cycloalkenyloxy group, an alkynyloxy group, an aryloxy group, a heterocyclic oxy group, an alkylthio group, a cycloalkylthio group, a cycloalkenylthio group, an alkynylthio group, an arylthio group, a heterocyclic thio group, an amino group, an alkylamino group, a cycloalkylamino group, an alkenylamino group, a cycloalkenylamino group, an arylamino group, a heterocyclic amino group, a silyl group, a silyloxy group or a halogen atom, and includes, preferably, an aryl group, a heterocyclic group, an alkoxy group, an aryloxy group, a heterocyclic oxy group, an alkylthio group, an arylthio group, a heterocyclic thio group, an amino group, an alkylamino group, an arylamino group and a heterocyclic amino group, and more preferably, an aryl group and a heterocyclic group. Specific examples of the aryl group include a phenyl group, a naphthyl group, an anthracenyl group and a pyrene ring group, preferably, a phenyl group and a naphthyl group, and particularly preferably, a phenyl group. Specific examples of heterocycles of the heterocyclic groups include a pyrrole ring, a pyridine ring, an indole ring, an imidazole ring, a benzimidazole ring, a pyrazole ring, a pyrazine ring, a pyrimidine ring, a pyridazine ring, a triazole ring, a benzotriazole ring, a tetrazole ring, an indazole ring, a triazine ring, a purine ring, a quinoline ring, an isoquinoline ring, a quinazoline ring, a thiazole ring, a benzothiazole ring, an isothiazole ring, an oxazole ring, an isoxazol ring, a furan ring, a benzo[b]furan ring, a thiophene ring and a benzo[b]thiophene ring, preferably, a pyrrole ring, a furan ring, a benzo[b]furan ring, a thiophene ring and a benzo[b]thiophene ring, and particularly preferably a thiophene ring and a

benzo[b]thiophene ring.

$Ar^1$ and $Ar^2$ preferably include an aromatic cyclic group among the groups specified. $Ar^1$ and $Ar^2$ preferably include, in a case of the heterocyclic group among the groups specified, one having a nitrogen atom in an annular atom, and one having at least one nitrogen atom on a 2-position (position adjacent to a position to be bonded with the pyridine ring A) of the cyclic group.

$Ar^1$ and $Ar^2$ preferably include a benzene ring group, a pyrrole ring group, an indole ring group, an imidazole ring group, a benzimidazole ring group, a pyrazole ring group, a benzopyrazole ring group (indazole ring group), a triazole ring group, a benzotriazole ring group, an oxazole ring group, a benzoxazole ring group, a thiazole ring group and a benzothiazole ring group.

All of $Ar^1$, $Ar^2$ and the pyridine ring A may have a substituent other than a carboxyl group, and when two or more substituents exist, these substituents may be bonded to form a ring.

Specific examples of the substituent other than the carboxyl group preferably include a substituent other than the below-mentioned acidic group. Specific examples of such substituents include a substituent T other than the below-mentioned carboxyl group, and preferably, other than the acidic group. Specific examples further preferably include an alkyl group, above all, a halogenated alkyl group (a perfluoroalkyl group such as a trifluoroalkyl group is preferred), and a halogen atom.

Specific examples of the rings formed by bonding two or more substituents with each other include a benzene ring and a heterocycle, and the heterocycle preferably includes an ethylenedioxy ring.

$Ar^1$ and $Ar^2$ preferably include a case where $Ar^1$ and $Ar^2$ are a ring group having ring structure different from each other, and when a coordinating atom of one of $Ar^1$ and $Ar^2$ incldues a carbon atom, a case where both are the above-described heterocyclic group having ring structure different from each other.

Moreover, $Ar^1$ and $Ar^2$ preferably include a case where $Ar^1$ and $Ar^2$ have, on at least one of $Ar^1$ and $Ar^2$, as a substituent, a halogen atom or a halogenated alkyl group (preferably, a chlorine atom-substituted or fluorine atom-substituted alkyl group, more preferably, a fluorine atom-substituted alkyl group, further preferably, a perfluoroalkyl group, and particularly preferably, a trifluoromethyl group).

Here, particularly in a benzene ring, a pyrazole ring or a triazole ring; a benzene ring, a pyrazole ring or a triazole ring is preferred in which substitution is made by an electron-withdrawing group (specific examples of the electron-withdrawing group include an electron-withdrawing group in the below-mentioned $R^{EWG}$) such as a halogen atom (particularly, a fluorine atom) and a perfluoroalkyl group (particularly, a perfluoromethyl group).

Among tridentate ligands represented by formula (A), a ligand represented by the following formula (A1) is preferred.

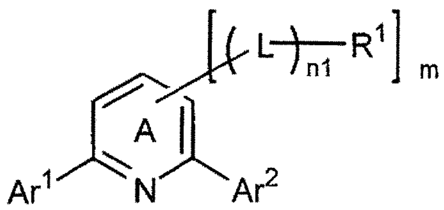

Formula (A1)

In the formula, $Ar^1$ and $Ar^2$ have definitions identical with the definitions in the formula (A), and a preferred range is also identical. Moreover, $-(L)n1-R^1$ has definitions identical with the definitions in the formula (Aa), and a preferred range is also identical.

m represents an integer of 0 to 3.

m is preferably 0 or 1.

The above-described $R^1$ preferably includes a group represented by the following formula (A1-1) or (A1-2).

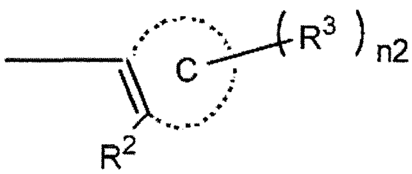

Formula (A1-1)

In the formula, a ring C represents an aromatic ring or a heteroaromatic ring. $R^2$ and $R^3$ each independently represent a substituent, provided that the formula (A1-1) does not represent the following formula (A1-2).

More specifically, the group represented by the formula (A1-1) essentially has a substituent $R^2$ on an ortho position of a bonding hand of the ring C, and the group represented by the formula (A1-2) has no substituent $R^2$ as in

the formula (A1-1) in a bonding hand of a furan ring, a pyrrole ring, a thiophene ring or a selenophene ring as formed by X.
n2 represents an integer of 0 to 4. When n2 is one or more, $R^2$ and $R^3$ may be bonded to form a ring, and when n2 is two or more, $R^3$ and $R^3$ may be bonded with each other to form a ring.
Specific examples of the rings formed by bonding these include a benzene ring, and a heterocycle such as an ethylenedioxy ring, a thiophene ring and a silacyclopentadiene ring.

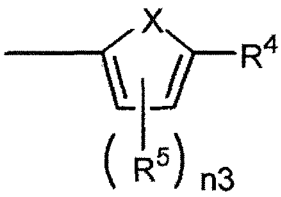

Formula (A1-2)

In the formula, X represents -O-, -N(Rz)-, -S- or -Se-.
$R^4$ represents a hydrogen atom, an alkyl group, an alkenyl group, an alkynyl group, an alkoxy group, an alkylthio group, an aryl group, an aryloxy group, an arylthio group, a silyloxy group, an amino group, a silyl group, a heterocyclic group or a halogen atom. Here, Rz represents an alkyl group, an alkenyl group, an alkynyl group, an aryl group, a silyl group or a heterocyclic group.
$R^5$ represents an alkyl group, an alkenyl group, an alkynyl group, an alkoxy group, an alkylthio group, an aryl group, an aryloxy group, an arylthio group, a silyloxy group, an amino group, a silyl group, a heterocyclic group or a halogen atom.
n3 represents an integer of 0 to 2, when n3 is one or more, $R^4$ and $R^5$ may be bonded to form a ring, and when n3 is 2, $R^5$ and $R^5$ may be bonded with each other to form a ring.
Specific examples of the rings formed by bonding these include a benzene ring, and a heterocycle such as an ethylenedioxy ring, a thiophene ring and a silacyclopentadiene ring.
In the formula (A1-1), specific examples of the aromatic ring of the ring C include a benzene ring, a naphthalene ring, an anthracene ring, a phenanthrene ring and a pyrene ring, and preferably, a benzene ring. Specific examples of the heteroaromatic ring include a pyrrole ring, a pyrazole ring, an imidazole ring, an oxazole ring, a thiazole ring, an isoxazol ring, an isothiazole ring, a furazan ring, a pyridine ring, a pyridazine ring, a pyrimidine ring, a pyrazine ring, an indole ring, an indazole ring, a benzopyrrole ring, an isoindole ring, a benzimidazole ring, a benzotriazole ring, a benzoxazole ring, a benzothiazole ring, a quinoline ring, an isoquinoline ring, a phthalazine ring, a cinnoline ring, a quinazoline ring, a phenanthridine ring, a phenanthroline ring, a quinoxaline ring, a naphthyridine ring, a pteridine ring, a β-carboline ring, a furan ring, a benzo[b]furan ring, a thiophene ring, a benzo[b]thiophene ring, a selenophene ring, a benzoselenophene ring and a triazine ring, and preferably, a pyrrole ring, a furan ring, a benzo[b]furan ring, a thiophene ring and a benzo[b]thiophene ring.
Here, the ring C includes, preferably, an aromatic ring, more preferably, a benzene ring, and further preferably, 2,6-disubstituted benzene ring group. In this case, n1 is preferably zero.
$R^2$ includes, preferably, an alkyl group, an alkoxy group, a cycloalkoxy group, an aryloxy group, a heterocyclic oxy group, an alkylamino group, an arylamino group and a heterocyclic amino group, more preferably, an alkyl group, an alkoxy group, a cycloalkoxy group, an aryloxy group and a heterocyclic oxy group, and further preferably, an alkoxy group.
$R^3$ includes, more preferably, an alkyl group, a cycloalkyl group, an aryl group, a heterocyclic group, an alkoxy group, a cycloalkyloxy group, an aryloxy group, a heterocyclic oxy group, an alkylthio group, an arylthio group, a heterocyclic thio group, an amino group, an alkylamino group, a cycloalkylamino group, an alkenylamino group, a cycloalkenylamino group, an arylamino group, a heterocyclic amino group and a halogen atom, further preferably, an alkyl group, a cycloalkyl group, an aryl group, a heterocyclic group, an alkoxy group, an aryloxy group, an alkylthio group, an arylthio group, an amino group, an alkylamino group, an arylamino group, a heterocyclic amino group and a halogen atom, and particularly preferably, an alkyl group, a cycloalkyl group, a heterocyclic group, an alkoxy group, an aryloxy group, an alkylthio group, an arylthio group, an amino group, an alkylamino group, an arylamino group, a heterocyclic amino group and a halogen atom.
n2 preferably includes an integer of 0 to 2, and further preferably, 0 or 1.
In the formula (A1-2), $R^4$ includes, preferably, an alkyl group, a cycloalkyl group, an alkenyl group, a cycloalkenyl group, an alkynyl group, a heterocyclic group, an alkoxy group, a cycloalkyloxy group, a cycloalkenyloxy group, an alkynyloxy group, an aryloxy group, a heterocyclic oxy group, an alkylthio group, a cycloalkylthio group, a cycloalkenylthio group, an alkynylthio group, an arylthio group, a heterocyclic thio group, an amino group, an alkylamino group, a cycloalkylamino group, an alkenylamino group, a cycloalkenylamino group, an arylamino group and a heterocyclic amino group, more preferably, an alkyl group, a cycloalkyl group, an alkenyl group, an alkynyl group, a heterocyclic group, an

alkoxy group, an aryloxy group, an alkylthio group, an arylthio group, an amino group, an alkylamino group, an arylamino group and a heterocyclic amino group, further preferably, an alkyl group, an alkenyl group, an alkynyl group or a heterocyclic group, and particularly preferably, an alkyl group.

X includes, preferably, -O-, -N(Rz)- and -S-, and more preferably, -S-.

$R^5$ includes, preferably, an alkyl group, an alkenyl group, an alkynyl group, an alkoxy group, an alkylthio group, an aryl group, an aryloxy group, an arylthio group and an amino group, and particularly preferably, an alkyl group, an alkenyl group, an alkoxy group, an aryl group and an aryloxy group.

Rz includes, preferably, an alkyl group and an aryl group, and particularly preferably, an alkyl group.

n3 is preferably 0 or 1. Specific examples of a ring formed by bonding a plurality of $R^5$ when n3 is two or more include 5-membered ring or a 6-membered ring, and these rings may be condensed. These rings include, preferably, a benzene ring, an ethylenedioxy ring and a heteroaryl ring, and more preferably, a benzene ring, an ethylenedioxy ring and a thiophene ring.

In the case of the group represented by the formula (A1-2), n1 includes, preferably, an integer of 1 to 3, more preferably, 1 or 2, and particularly preferably 1.

In the formulas (A1-1) and (A1-2), $R^2$ preferably includes an alkoxy group having 6 to 12 carbons atoms, a cycloalkoxy group, an aryloxy group and a heterocyclic oxy group, and $R^4$ preferably includes an alkyl group having 6 to 12 carbon atoms, a cycloalkyl group, an alkenyl group, a cycloalkynyl group and an alkynyl group.

Specific examples of the ligand LD are shown below, but the present invention is not limited thereby.

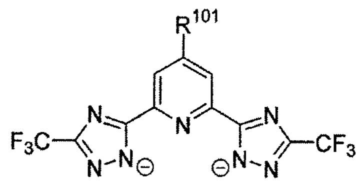

| | $R^{101}$ |
|---|---|
| LD-1-1 | 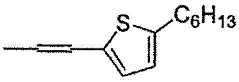 |
| LD-1-2 | 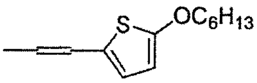 |
| LD-1-3 | 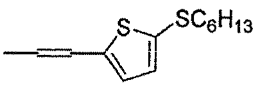 |
| LD-1-4 | 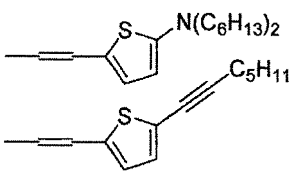 |
| LD-1-5 | |
| LD-1-6 | 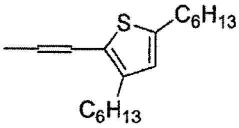 |
| LD-1-7 | 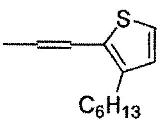 |
| LD-1-8 | 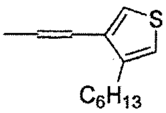 |
| LD-1-9 | 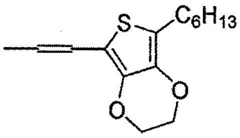 |
| LD-1-10 | 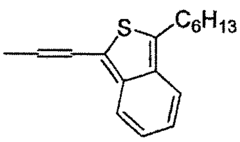 |
| LD-1-11 | 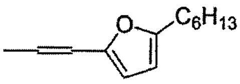 |
| LD-1-12 | 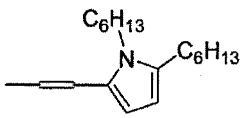 |

| | $R^{101}$ |
|---|---|
| LD-1-13 | 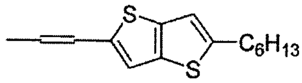 |
| LD-1-14 | 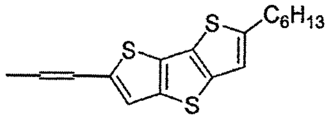 |
| LD-1-15 | 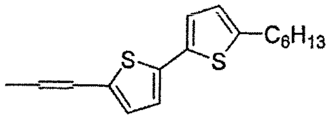 |
| LD-1-16 | 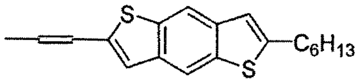 |
| LD-1-17 | 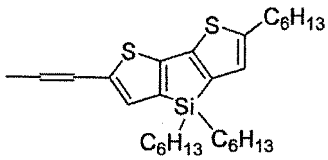 |
| LD-1-18 | 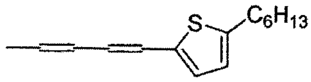 |
| LD-1-19 | 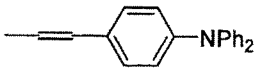 |
| LD-1-20 | 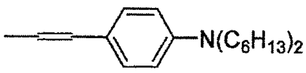 |
| LD-1-21 | 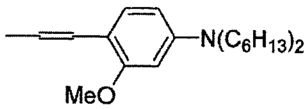 |
| LD-1-22 | 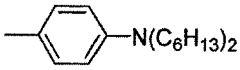 |
| LD-1-23 | 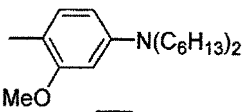 |
| LD-1-24 | 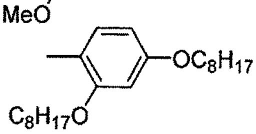 |
| LD-1-25 | 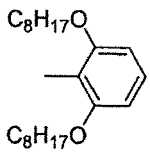 |

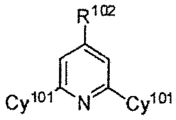

| | $Cy^{101}$ / $R^{102}$ |
|---|---|
| LD-2-1 | 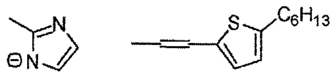 |
| LD-2-2 | 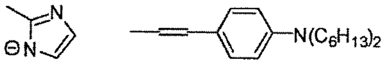 |
| LD-2-3 | 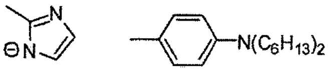 |
| LD-2-4 | 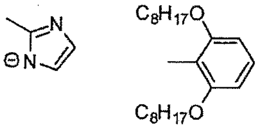 |
| LD-2-5 | 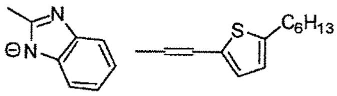 |
| LD-2-6 | 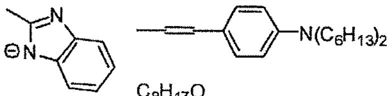 |
| LD-2-7 | 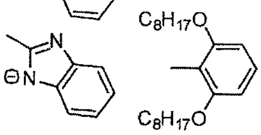 |
| LD-2-8 | 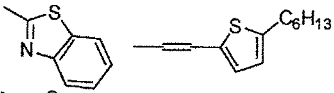 |
| LD-2-9 | 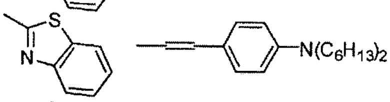 |
| LD-2-10 | 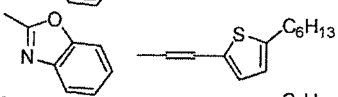 |
| LD-2-11 | 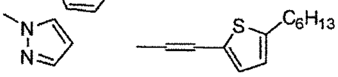 |
| LD-2-12 | 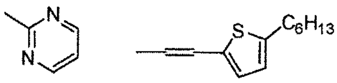 |
| LD-2-13 | 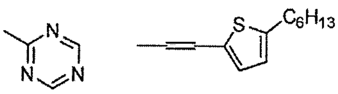 |
| LD-2-14 | 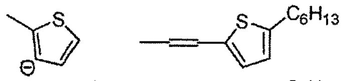 |
| LD-2-15 | 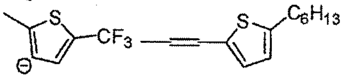 |
| LD-2-16 | 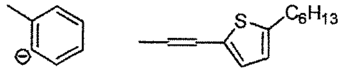 |
| LD-2-17 | 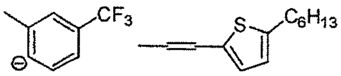 |
| LD-2-18 | 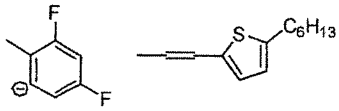 |
| LD-2-19 | 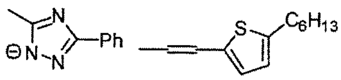 |
| LD-2-20 | 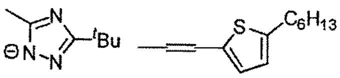 |
| LD-2-21 | 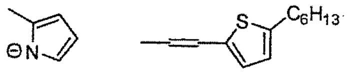 |
| LD-2-22 | 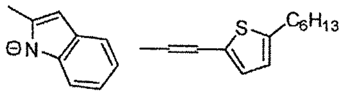 |
| LD-2-23 | 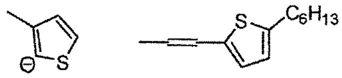 |
| LD-2-24 | 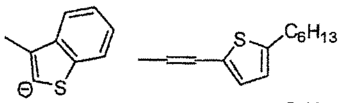 |
| LD-2-25 | 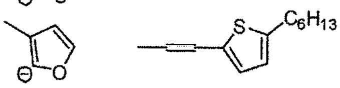 |
| LD-2-26 | 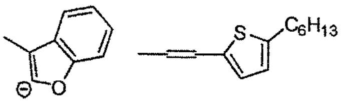 |

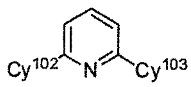

| | $Cy^{102}$ | $Cy^{103}$ |
|---|---|---|
| LD-3-1 | 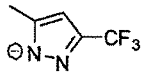 | 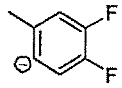 |
| LD-3-2 | 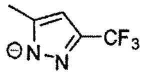 | 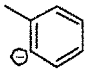 |
| LD-3-3 | 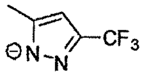 | 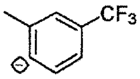 |
| LD-3-4 | 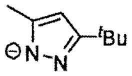 | 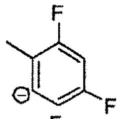 |
| LD-3-5 | 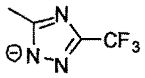 | 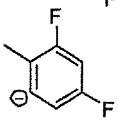 |
| LD-3-6 | 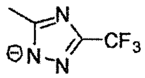 | 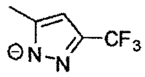 |
| LD-3-7 | 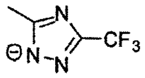 | 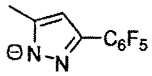 |

| | $Cy^{102}$ | $Cy^{103}$ |
|---|---|---|
| LD-3-8 | 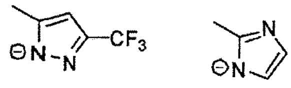 | |
| LD-3-9 | 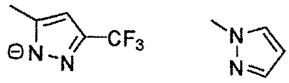 | |
| LD-3-10 | 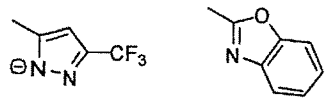 | |
| LD-3-11 | 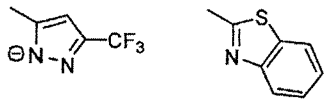 | |
| LD-3-12 | 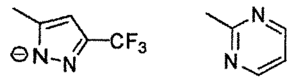 | |
| LD-3-13 | 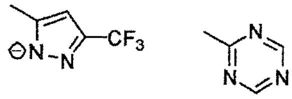 | |
| LD-3-14 | 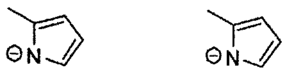 | |
| LD-3-15 | 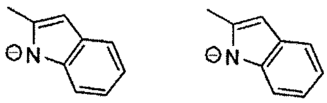 | |
| LD-3-16 | 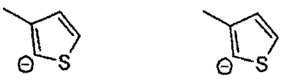 | |
| LD-3-17 | 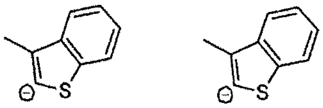 | |
| LD-3-18 | 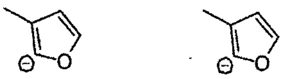 | |
| LD-3-19 | 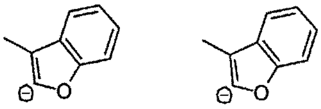 | |

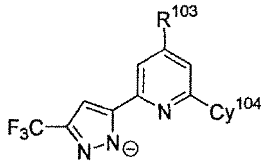

| | Cy104 | R103 | | Cy104 | R103 |
|---|---|---|---|---|---|
| LD-4-1 | 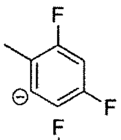 | 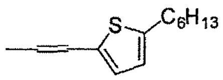 | LD-4-10 | 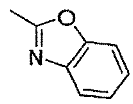 | 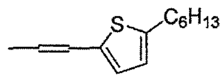 |
| LD-4-2 | 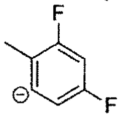 | 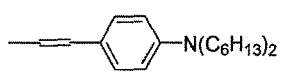 | LD-4-11 | 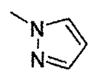 | 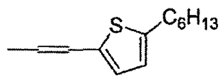 |
| LD-4-3 | 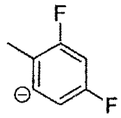 | 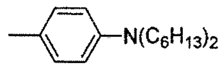 | LD-4-12 | 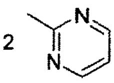 | 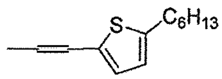 |
| LD-4-4 | 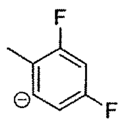 | 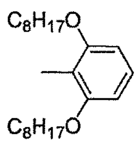 | LD-4-13 | 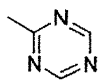 | 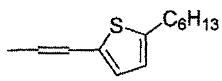 |
| LD-4-5 | 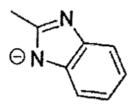 | 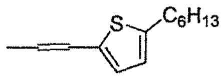 | LD-4-14 | 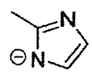 | 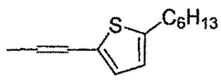 |
| LD-4-6 | 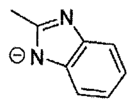 | 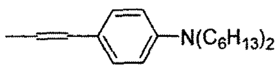 | LD-4-15 | 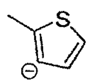 | 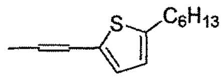 |
| LD-4-7 | 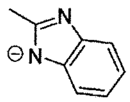 | 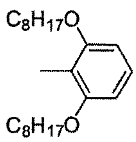 | LD-4-16 | 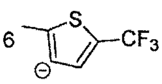 | 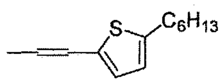 |
| LD-4-8 | 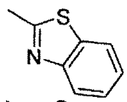 | 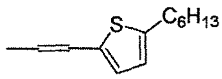 | LD-4-17 | 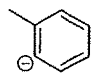 | 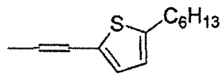 |
| LD-4-9 | 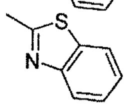 | 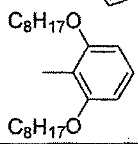 | LD-4-18 | 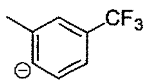 | 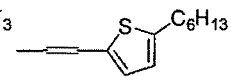 |

In the above, "Me" represents a methyl group, "tBu" represents a t-butyl group, and "Ph" represents a phenyl group ($-C_6H_5$).

The ligand LD represented by formula (A) can be readily synthesized by methods described in US 2010/0258175 A1, Japanese Patent No. 4298799, and Angew. Chem. Int. Ed., 2011, 50, 2054-2058, methods described in references cited in the literatures, or methods according to these methods.

<Ligand LA>

In the present invention, the ligand LA is categorized as an acceptor ligand, and is represented by formula (B).

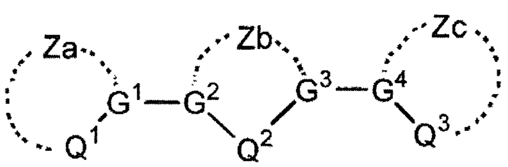 Formula (B)

In formula, Za, Zb and Zc each independently represent a group of atoms for forming a 5- or 6-membered ring, in which at least one of the rings formed by Za, Zb and Zc has an acidic group.

$Q^1$ to $Q^3$ each independently represent a nitrogen atom having a lone electron pair, a nitrogen atom having an anion, or a carbon atom having an anion.

$G^1$ to $G^4$ each independently represent a carbon atom or a nitrogen atom.

The ring formed by Za, Zb and Zc includes a ring having a carbon anion in an annular atom or a nitrogen-containing heterocycle having a nitrogen atom or nitrogen anion having a lone electron pair in the annular atom, and these rings may be condensed.

In the above, a bond between $Q^1$-$G^1$, a bond between $G^2$-$Q^2$, a bond between $G^3$-$Q^2$, and a bond between $G^4$-$Q^3$ are indicated by "-" for the sake of convenience. The bonds between these atoms indicate that these atoms bind to one another, and the bond may be a single bond (-) or a double bond (=).

The rings formed by Za, Zb and Zc may be either an aromatic ring or a ring other than an aromatic ring, and an aromatic ring (an aromatic ring or a heteroaromatic ring) is preferred.

Specific examples of rings of the rings having the carbon anion in the annular atom include a cyclopentadiene ring, a benzene ring, a naphthalene ring, a pyrrole ring, an oxazole ring, a thiazole ring, an isoxazol ring, an isothiazole ring, a pyridine ring, a pyridazine ring, an indole ring, an indazole ring, a benzopyrrole ring, an isoindole ring, a benzimidazole ring, a benzotriazole ring, a benzoxazole ring, a benzothiazole ring, a quinoline ring, an isoquinoline ring, a phthalazine ring, a cinnoline ring, a quinazoline ring, a phenanthridine ring, a phenanthroline ring, a quinoxaline ring, a naphthyridine ring, a β-carboline ring, a furan ring, a benzo[b]furan ring, a thiophene ring and a benzo[b]thiophene ring, and preferably, a benzene ring, a furan ring, a benzo[b]furan ring, a thiophene ring and a benzo[b]thiophene ring.

A ring of the nitrogen-containing heterocycle having the nitrogen atom or nitrogen anion having the lone electron pair in the annular atom preferably includes one having only a nitrogen atom in the annular atom and one containing an oxygen atom or a sulfur atom together with the nitrogen atom.

Examples of the nitrogen-containing heteroaromatic ring include a pyrrole ring, a pyrazole ring, an imidazole ring, a triazole ring, a tetrazole ring, an oxazole ring, a thiazole ring, an isoxazole ring, an isothiazole ring, a furazan ring, a pyridine ring, a pyridazine ring, a pyrimidine ring, a pyrazine ring, a triazine ring, an indole ring, a benzopyrrol ring, an isoindole ring, a benzimidazole ring, a benzotriazole ring, a benzoxazole ring, a benzothiazole ring, a quinoline ring, an isoquinoline ring, a phthalazine ring, a cinnoline ring, a quinazoline ring, a phenanthridine ring, a phenanthroline ring, a quinoxaline ring, a naphthyridine ring, a purine ring, a pteridine ring, and a β-carboline ring. Examples of the nitrogen-containing hetero ring other than the heteroaromatic ring includes a pyrrolidine ring, a pyrroline ring, a pyrazoline ring, a pyrazoline ring, an imidazolidine ring, an imidazoline ring, a piperazine ring, a piperidine ring, a morpholine ring, an indoline ring, and an isoindoline ring.

The ring formed by Za, Zb and Zc includes, preferably, a ring of the nitrogen-containing heterocycle having the nitrogen atom or nitrogen anion having the lone electron pair in the annular atom, and more preferably, a pyridine ring, a pyrimidine ring, a benzothiazole ring, a quinoline ring and an isoquinoline ring.

At least one of the rings formed by Za, Zb and Zc has an acidic group. The number of acidic groups is preferably from 1 to 6, more preferably from 1 to 4, and further preferably from 1 to 3.

The acidic group represents a substituent having a dissociative proton. Examples thereof include: an acid group which shows an acid property, such as a carboxyl group, a phosphonyl group, a phosphoryl group, a sulfo group, and a boric acid group; or a group having any of these groups. Further, the acidic group may be in a dissociation form due to release of a proton, or may be a salt thereof. The acidic group includes, preferably, any one of a carboxy group, a sulfo group, a phosphonyl group or a phosphoryl group and a salt thereof, and particularly preferably, a carboxy group and a group having the same.

The acidic group may be substituted directly at the ring formed by any of Za to Zc, or may be a group which binds to the ring via a linking group.

When the acid group includes the group through the linking group, specific examples of the linking groups include an alkylene group, an alkylene group, an alkenylene group, an arylene group, a divalent heterocyclic group and a group in which these groups are combined. As the alkylene group, the number of carbon atoms is preferably 1 to 4, as the alkenylene group, the number of carbon atoms is preferably 2 to 4, as the alkynylene group, the number of carbon atoms is preferably 2 to 4, as the arylene group, the number of carbon atoms is preferably 6 to 12, and as the divalent heterocyclic group, the number of carbon atoms is preferably 0 to 12, respectively.

Specific examples of the group through the linking group include a carboxyvinylene group, a dicarboxyvinylene group, a cyanocarboxyvinylene group and a carboxyphenyl group as a preferred one.

When the acidic group is a salt, a counter ion upon forming the salt is not particularly limited, and specific examples include a positive ion in the below-mentioned counter ion Y.

In the present invention, from a viewpoint of electron transfer, one in which the acid group is directly bonded with the above-described ring is more preferred to the group through the linking group according to the present invention.

In a case where the ring formed by any of Za to Zc is a pyridine ring, the pyridine ring having an acidic group

at the p-position with respect to the nitrogen atom thereof is preferred.

The group represented by Za, Zb or Zc may be have a substituent other than the acidic group, and specific examples of these substituents include the substituent T described below.

$G^1$ to $G^4$ each represent a carbon atom or a nitrogen atom, and each of $G^2$ and $G^3$ is preferably a carbon atom. In a case where each of $G^2$ and $G^3$ is a carbon atom, $G^1$ and/or $G^4$ are preferably a nitrogen atom. In the present invention, it is particularly preferred that all of $G^1$ to $G^4$ are a carbon atom.

In a case where $G^1$ and/or $G^4$ is or are a nitrogen atom, the ring to be formed by Za and $Q^1$, and/or Zc and $Q^4$ is preferably a pyrazole ring, or a triazole ring.

The ligand LA represented by formula (B) is preferably a compound represented by any one of formulas (B1) to (B8).

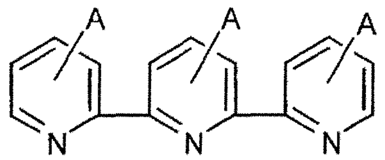

Formula (B1)

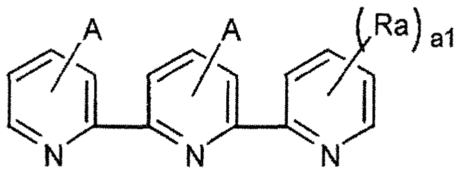

Formula (B2)

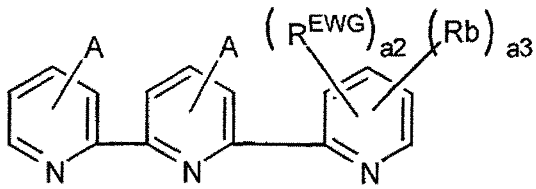

Formula (B3)

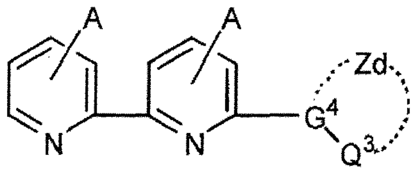

Formula (B4)

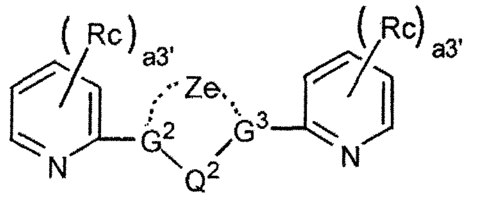

Formula (B5)

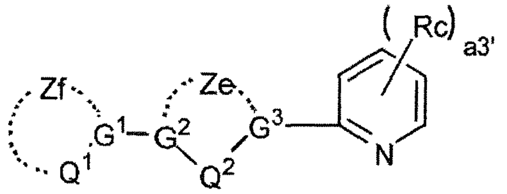

Formula (B6)

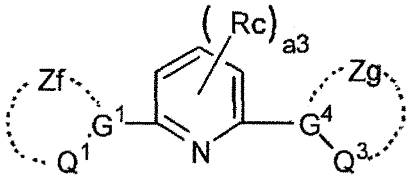

Formula (B7)

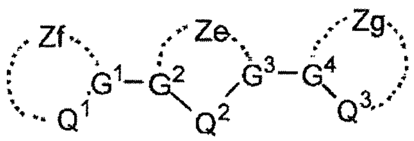

Formula (B8)

In formulas, $Q^1$ to $Q^3$ and $G^1$ to $G^4$ have the same meaning as those in formula (B), respectively;
Zd represent a group of atom for forming a benzene ring, a naphthalene ring, an anthracene ring, a phenanthrene ring, a pyrene ring, a perylene ring, a pyrrole ring, an indole ring, an imidazole ring, a benzimidazole ring, a pyrazole ring, a pyrazine ring, a pyrimidine ring, a pyridazine ring, a triazole ring, a benzotriazole ring, a tetrazole ring, an indazole ring, a triazine ring, a purine ring, a quinoline ring, an isoquinoline ring, a quinazoline ring, a thiazole ring, a benzothiazole ring, an isothiazole ring, an oxazole ring, an isoxazole ring, a furan ring, a benzo[b]furan ring, a thiophene ring, a benzo[b]thiophene ring, a pyrrolidine ring, a piperidine ring, a morpholine ring, a piperazine ring, a tetrahydrofuran ring, a tetrahydropyran ring, a 4H-pyran ring, a 1,4-dihydropyridine ring, a tetradehydromorpholine ring, or a tetradehydrothiomorpholine ring.

The ring formed by Zd is preferably an aromatic ring. The aromatic ring is preferably a benzimidazole ring, a

benzothiazole ring, a thiophene ring, a pyrrole ring, a pyrazole ring, a triazole ring, a pyrimidine ring, a pyridazine ring, a pyrazine ring, a quinoline ring, or a benzene ring; more preferably a benzothiazole ring, a benzimidazole ring, a quinoline ring, a pyrazole ring, a triazole ring, or a benzene ring; particularly preferably a benzothiazole ring, a benzimidazole ring, a triazole ring, a triazine ring or a quinoline ring; and most preferably a quinoline ring.

Ze to Zg represent a group of atom for forming a 5 - or 6-membered ring other than a pyridine ring.

A represents an acidic group.

Ra represents an aryl group, a heteroaryl group, an alkyl group, a cycloalkyl group, an alkoxy group, or a cycloalkoxy group; in which the alkyl group has a tertiary or quaternary carbon atom, and the alkoxy group has a tertiary or quaternary carbon atom, or a carbon atom directly binding to an oxygen atom of the alkoxy group is a secondary or tertiary carbon atom.

Ra is preferably an aryl group, a heteroaryl group, or an alkyl group, more preferably an aryl group, or a heteroaryl group, and particularly preferably a heteroaryl group. If Ra is bulky, association of the metal complex dyes is conceivably suppressed, and therefore to cause efficient progress of electron injection into an oxide semiconductor and to improve the short-circuit current density (Jsc).

As the aryl group, the number of carbon atoms is preferably 6 to 20, more preferably, 6 to 16, particularly preferably, 6 to 14, and an aryl group having 6 to 10 carbon atoms is most preferred, and a benzene ring is still most preferred.

As the heteroaryl group, the number of atoms other than a hydrogen atom is preferably 5 to 30, more preferably, 5 to 25, further preferably, 5 to 20, and particularly preferably, 5 to 12, and a thiophene ring is most preferred.

a1 represents an integer of 1 or 2.

$R^{EWG}$ represents an electron-withdrawing group. $R^{EWG}$ preferably includes an electron-withdrawing group that is not an acidic group including a carboxyl group.

The electron-withdrawing group is further described in below.

Examples of the electron-withdrawing group include a substituent having the -I effect or the -M effect.

In general, an electron-withdrawing group attenuates the electron density at a particular position of a molecule. The electron-withdrawing property or electron-donating property cannot be explained only by the difference in the electronegativity. That is, since an inductive effect, a mesomeric effect and the like work together in a composite manner, the manifestation of the electron-withdrawing property or the electron-donating property can vary with the aromaticity, presence of a conjugated system, or a topological positional relationship. As an experimental rule for quantitatively evaluating and predicting these effects on the basis of the acid dissociation constant of para- and meta-substituted benzoic acid, there is known Hammett's rule. In the case of the inductive effect, the electron-withdrawing effect is referred to as the -I effect, while the electron-donating effect is referred to as the +I effect, and an atom having higher electronegativity than carbon exhibits the -I effect. Furthermore, an anion exhibits the +I effect, while a cation exhibits the -I effect. In the case of the mesomeric effect, the electron-withdrawing effect is referred to as the -M effect, while the electron-donating effect is referred to as the +M effect. Examples of the electron-withdrawing group are shown below.

Inductive effect
(-I effect)

- $-O^+R_2 > -N^+R_3$
- $-N^+R_3 > -P^+R_3 > ...$
- $-O^+R_2 > -S^+R_2 > ...$
- $-N^+R_3 > -NO_2 > -SO_2R > -SOR$
- $-SO_2R > -SO_3R$
- $-N^+R_3 > -NR_2$
- $-O^+r_2 > -OR$
- $-S^+R_2 > -SR$
- $-F > -Cl > -Br > -I$
- $=O > =NR > =CR_2$
- $=O > -OR$
- $\equiv N > \equiv CR$
- $\equiv N > =NR > -NR_2$
- $-C \equiv CR > -CR=CR_2 > -CR_2CR_3$

Mesomeric effect
(-M effect)

- $=N^+R_3 > =NR$
- $=O > =NR > =CR_2$

- =S > =O > ≡N

In the above, R represents a substituent, and is representatively an alkyl group. The group may bind directly, or may bind via a conjugated system, for example, an aromatic ring, an ethynyl group, or an ethenyl group.

Examples of the case of binding via an aromatic ring include a halogenated phenyl group, a cyanopheny group, and a trifluoromethylphenyl group.

Further, of the substituent T described below, groups with σp of 0 or more according to Hammett's rule are enumerated as a specific group. Electron-withdrawing groups other than an acidic group are preferred, and preferable examples thereof include a fluoroalkyl group (for example, a perfluoroalkyl group, such as trifluoromethyl) and a halogen atom.

Rb and Rc represent a substituent. Specific examples of such a substituent include the substituent T described below.

a2 represents an integer of 1 to 4, a3 represents an integer of 0 to 3, and a3' represents an integer of 0 to 4. The sum of a2 and a3 is an integer of 1 to 4.

The ligands represented by any of formulas (B5) to (B8) each have at least one (1) acidic group.

Here, in Ra in the formula (B2), specific examples of the aryl group include phenyl and naphthyl, and preferably, phenyl that may have a substituent, and the heteroaryl group preferably includes the heteroaryl ring of the groups listed in $Ar^1$ or $Ar^2$ in the formula (A). Specific examples of the cycloalkyl group include cyclopentyl and cyclohexyl, and specific examples of the cycloalkoxy group include cyclopentyloxy and cyclohexyloxy.

"Tertiary carbon atom" means one in which any carbon atom in an alkyl group has one hydrogen atom, and "quaternary carbon atom" means one in which all of four bonding hands are bonded with a carbon atom.

Specific examples of the alkyl group having the tertiary carbon atom include an isoalkyl group that is branched into two at a terminal, such as an isobutyl group, and specific examples of the alkyl group having the quaternary carbon atom include a group having a t-alkyl group on and after a 2-position of an alkyl group, such as 2,4,4-trimethyl pentyl.

From a viewpoint of association prevention due to bulkiness, the alkyl group preferably includes one containing a quaternary carbon atom. Moreover, the number of carbon atoms of the alkyl group is preferably 4 to 30, more preferably, 6 to 28, further preferably, 6 to 26, and particularly preferably, 6 to 20.

Specific examples include t -butyl, i-pentyl, isohexyl, isooctyl, 2-ethylhexyl, adamantyl, isodecyl, isodecyl and isooctacosyl.

Specific examples of ones in which the alkoxy group has the tertiary or quaternary carbon atom include an alkoxy group having the above-described alkyl group, and specific examples of the alkoxy group in which the carbon atom directly bonded with the oxygen atom of the alkoxy group includes the secondary or tertiary carbon atom include a s-alkyloxy group and a t-alkyloxy group, and include i-propoxy, i-butoxy, t-butoxy, i-pentoxy, i-octyloxy, 2-ethylhexyloxy, isodecyl oxy, isohexadecyloxy and isooctacosyloxy.

The number of carbon atoms of the alkoxy group is preferably 3 to 30, more preferably, 4 to 30, further preferably, 4 to 26, and particularly preferably, 4 to 20.

Of the ligands represented by any of formulae (B1) to (B8), the ligand represented by formula (B1) is preferred, from the viewpoint of adsorption power to the semiconductor fine-particle surface. The ligand represented by formula (B2) is preferred, from the viewpoint of short-circuit current density (Jsc). The ligand represented by formula (B3) or (B4) is preferred, from the viewpoint of solution stability of the resultant metal complex dye. The compounds represented by any of formulae (B5) to (B8) are preferred, from the viewpoint of adsorption rate from the semiconductor fine-particle surface.

Specific examples of the ligand LA are shown below, but the present invention is not limited to these.

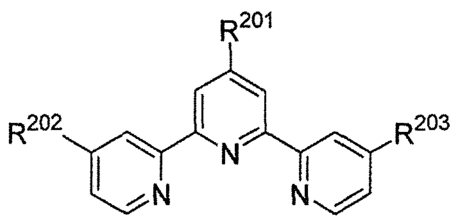

| | $R^{201}$ | $R^{202}$ | $R^{203}$ |
|---|---|---|---|
| LA-1-1 | -COOH | -COOH | -COOH |
| LA-1-2 | -COOH | -COOH | -H |
| LA-1-3 | -COOH | -H | -COOH |
| LA-1-4 | -H | -COOH | -H |

(continued)

| | $R^{201}$ | $R^{202}$ | $R^{203}$ |
|---|---|---|---|
| LA-1-5 | -H | $-PO_3H_2$ | -H |
| LA-1-6 | -H | $-SO_3H$ | -H |

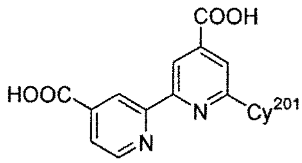

| | $Cy^{201}$ | | $Cy^{201}$ | | $Cy^{201}$ | | $Cy^{201}$ |
|---|---|---|---|---|---|---|---|
| LA-2-1 | 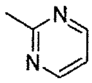 | LA-2-9 | 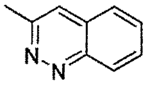 | LA-2-17 | 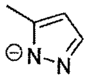 | LA-2-25 | 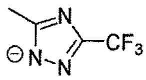 |
| LA-2-2 | 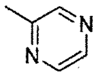 | LA-2-10 | 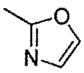 | LA-2-18 | 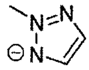 | LA-2-26 | 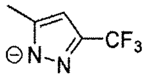 |
| LA-2-3 | 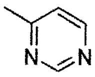 | LA-2-11 | 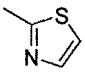 | LA-2-19 | 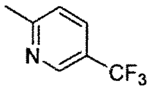 | LA-2-27 | 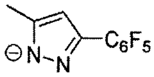 |
| LA-2-4 | 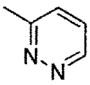 | LA-2-12 | 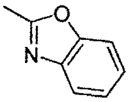 | LA-2-20 | 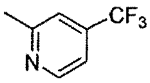 | LA-2-28 | 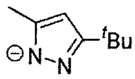 |
| LA-2-5 | 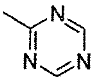 | LA-2-13 | 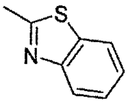 | LA-2-21 | 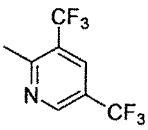 | LA-2-29 | 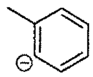 |
| LA-2-6 | 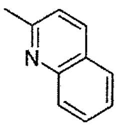 | LA-2-14 | 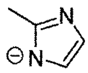 | LA-2-22 | 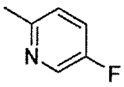 | LA-2-30 | 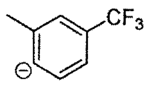 |
| LA-2-7 | 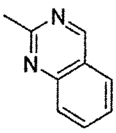 | LA-2-15 | 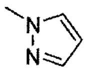 | LA-2-23 | 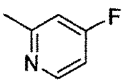 | LA-2-31 | 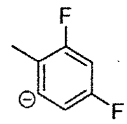 |
| LA-2-8 | 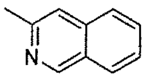 | LA-2-16 | 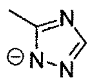 | LA-2-24 | 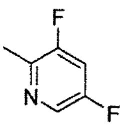 | | |

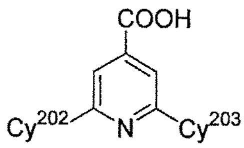

| | $Cy^{202}$ | $Cy^{203}$ |
|---|---|---|
| LA-3-1 | 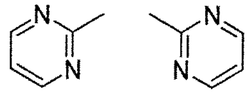 | |
| LA-3-2 | 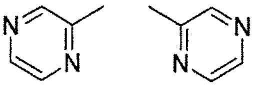 | |
| LA-3-3 | 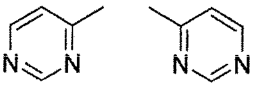 | |
| LA-3-4 | 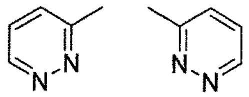 | |
| LA-3-5 | 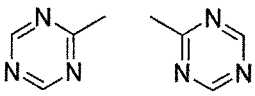 | |
| LA-3-6 | 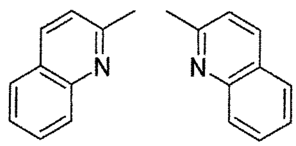 | |
| LA-3-7 | 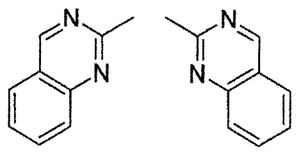 | |
| LA-3-8 | 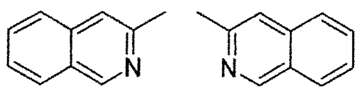 | |
| LA-3-9 | 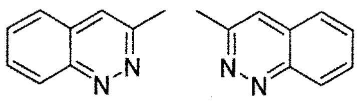 | |
| LA-3-10 | 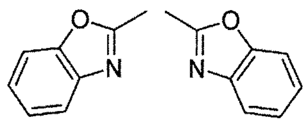 | |
| LA-3-11 | 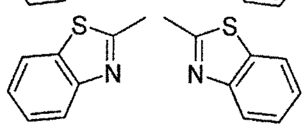 | |

| | $Cy^{202}$ | $Cy^{203}$ |
|---|---|---|
| LA-3-12 | 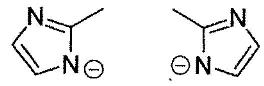 | |
| LA-3-13 | 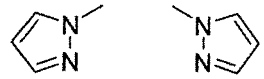 | |
| LA-3-14 | 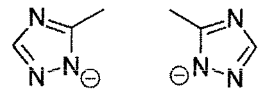 | |
| LA-3-15 | 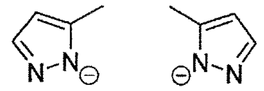 | |
| LA-3-16 | 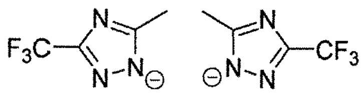 | |
| LA-3-17 | 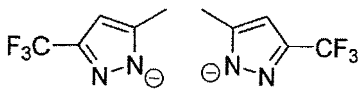 | |
| LA-3-18 | 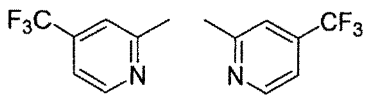 | |
| LA-3-19 | 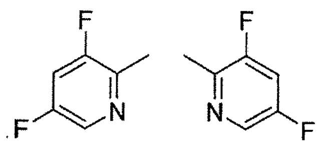 | |
| LA-3-20 | 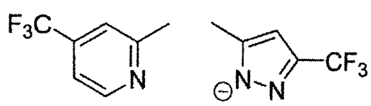 | |
| LA-3-21 | 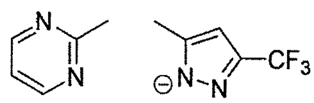 | |

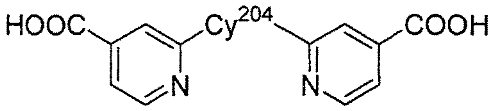

| | $Cy^{204}$ | | $Cy^{204}$ |
|---|---|---|---|
| LA-4-1 | 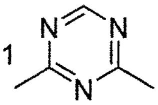 | LA-4-4 | 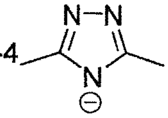 |
| LA-4-2 | 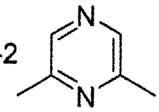 | LA-4-5 | 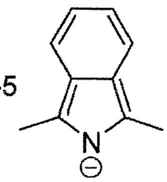 |
| LA-4-3 | 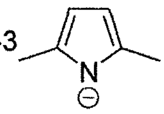 | | |

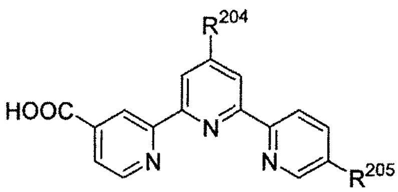

| | $R^{204}$ | $R^{205}$ | | $R^{204}$ | $R^{205}$ |
|---|---|---|---|---|---|
| LA-5-1 | -COOH | 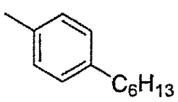 | LA-5-5 | 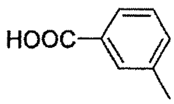 | 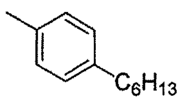 |
| LA-5-2 | -COOH | 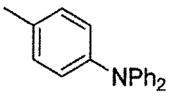 | LA-5-6 | 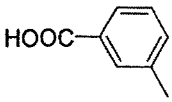 | 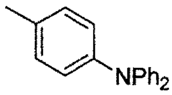 |
| LA-5-3 | -COOH | 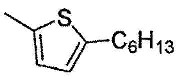 | LA-5-7 | 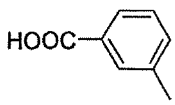 | 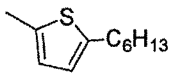 |
| LA-5-4 | -COOH | 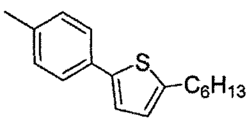 | LA-5-8 | 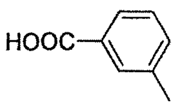 | 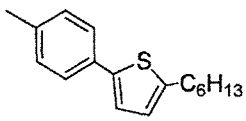 |

In the above, "tBu" represents a t-butyl group, and "Ph" represents a phenyl group ($-C_6H_5$).

The ligand LA represented by formula (B) can be readily synthesized in the same manner as the ligand LD represented by formula (A).

<Counter ion Y>

Counter ion Y represents a counter ion in the case where the counter ion is necessary to neutralize a charge. Generally, whether the metal complex dye is cationic or anionic, or has a net ionic charge, depends on the metal, the ligand and the substituent, in the metal complex dye.

In the case where the substituent has a dissociative group or the like, the metal complex dye represented by formula (I) may have a negative charge arising from dissociation. In this case, an electric charge of the metal complex dye represented by formula (I) as a whole is electrically neutralized by the counter ion Y.

When the counter ion Y is a positive counter ion, examples of the counter ion Y include an inorganic or organic ammonium ion (for example, tetraalkyl ammonium ion, pyridinium ion, and the like), a phosphonium ion (for example, a tetralkylphosphonium ion, an alkyltriphenylphosphonium ion, and the like), an alkali metal ion, and a hydrogen ion (a proton).

When the counter ion Y is a negative counter ion, the counter ion Y may be an inorganic negative ion or an organic negative ion. Examples thereof include a halogen ion (for example, fluoride ion, chloride ion, bromide ion, iodide ion), an arylsulfonate ion (for example, p-toluene sulfonate ion, p-chlorobenzene sulfonate ion), an aryldisulfonate ion (for example, 1,3-benzene disulfonate ion, 1,5-naphthalene disulfonate ion, 2,6-naphthalene disulfonate ion), an alkylsulfate ion (for example, methylsulfate ion), a sulfate ion, a thiocyanate ion, a perchlorate ion, a tetrafluoroborate ion, a hexafluorophosphae ion, a picrate ion, an acetate ion, and a trifluoromethane sulfonate ion. Alternatively, as a charge balance counter ion, an ionic polymer or another dye with the opposite charge from the dye in interest may be used. Alternatively, a metal complex ion (for example, bisbenzene-1,2-dithiolatonickel (III) and the like) may be used.

In the present invention, Y is preferably a halogen ion, an arylsulfonate ion, an aryldisulfonate ion, an alkylsulfate ion, a sulfate ion, a thiocyanate ion, a perchlorate ion, a tetrafluoroborate ion, a hexafluorophosphate ion, an acetate ion, a trifluoromethanesulfonate ion, an ammonium ion, an alkali metal ion or a hydrogen ion.

mY represents an interger of 0 to 2.

<Metal complex dye represented by formula (I)>

Specific examples of the metal complex dye represeted by formula (I) of the present invention are shown below, but the present invention is not limited to these.

Dye 1

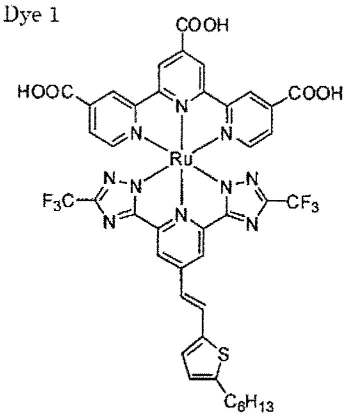

Dye 2

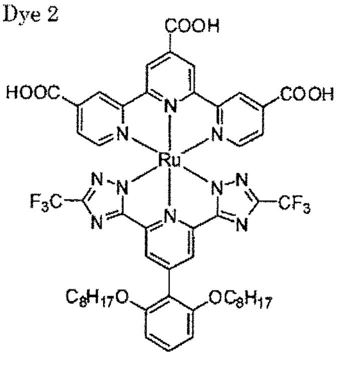

Dye 3

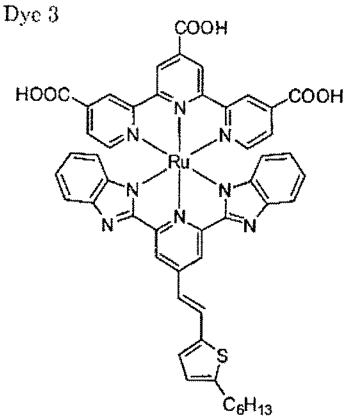

Dye 4

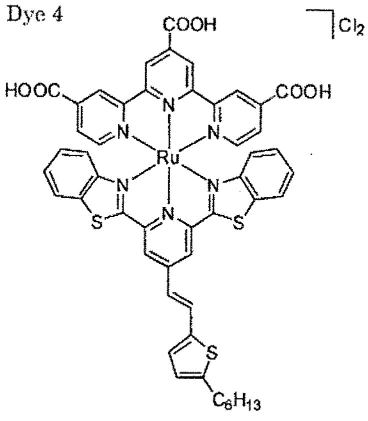

Dye 5

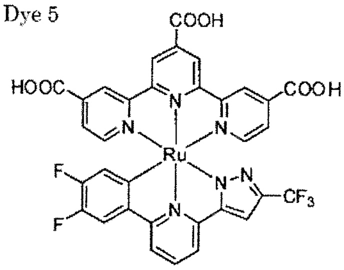

Dye 6

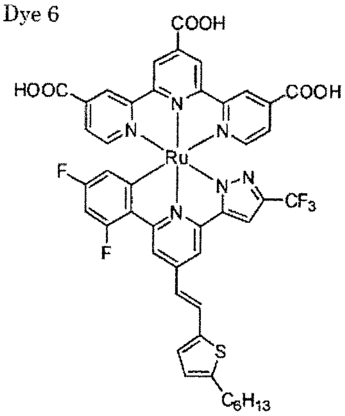

Dye 7

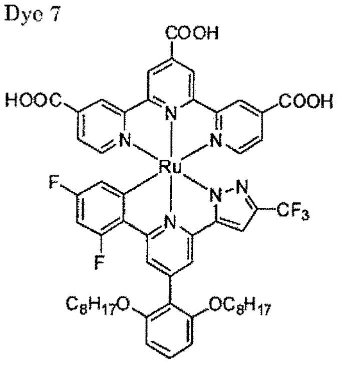

Dye 8

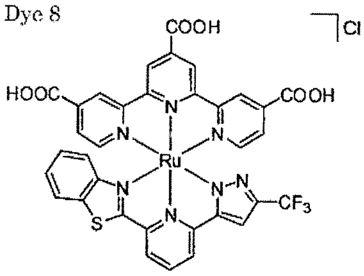

Dye 9

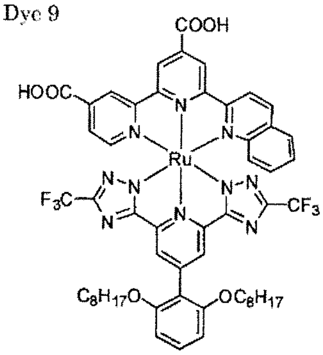

Dye 10

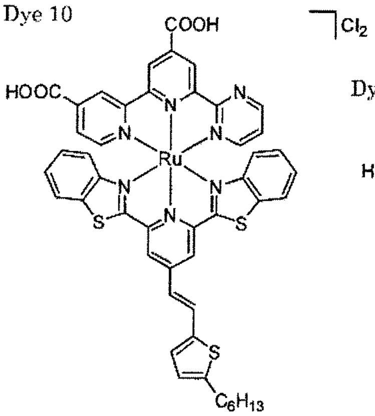

Dye 11

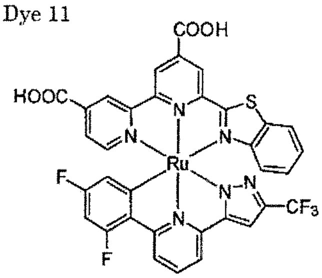

Dye 12

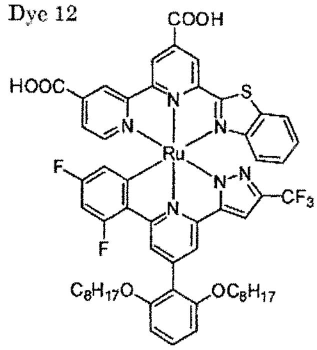

Dye 13

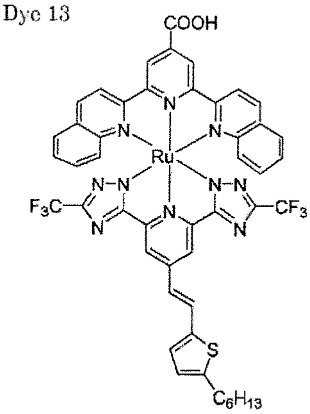

Dye 14

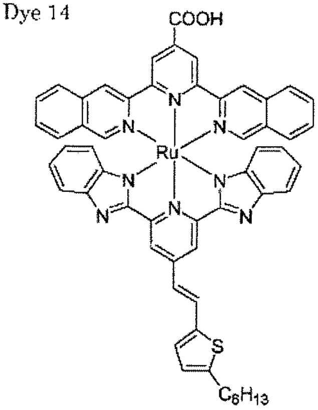

Dye 15

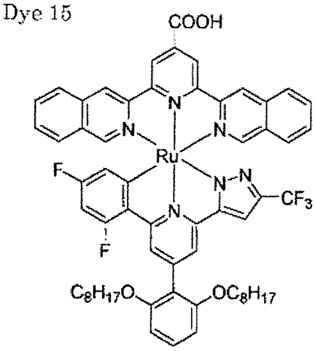

Dye 16

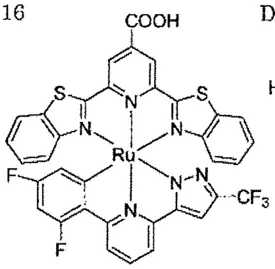

Dye 17

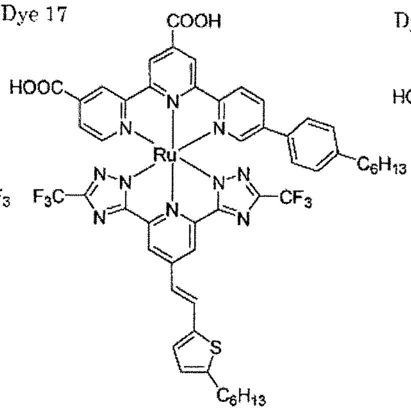

Dye 18

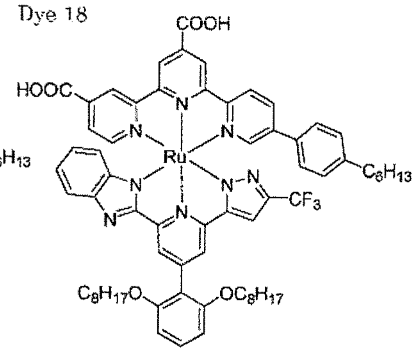

Dye 19

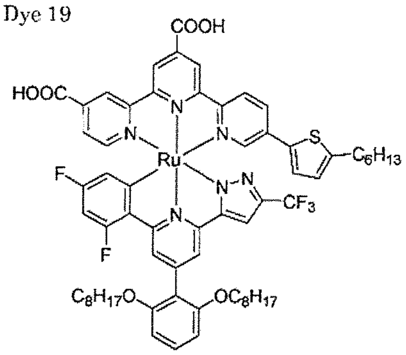

Dye 20

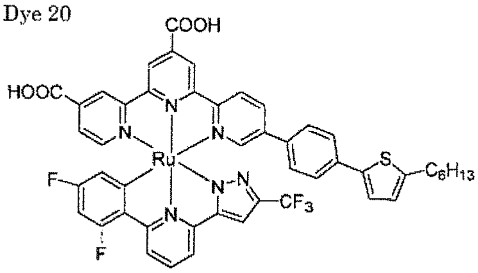

Dye 21

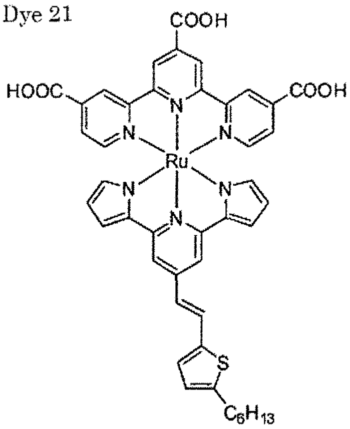

Dye 22

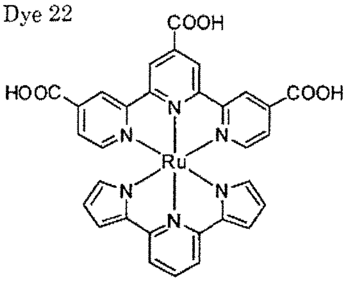

Dye 23

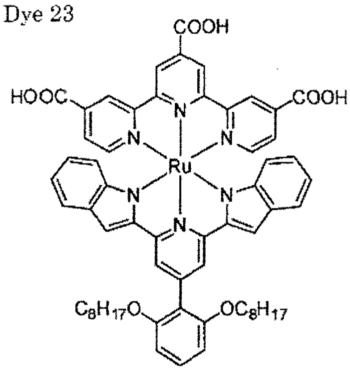

Dye 24

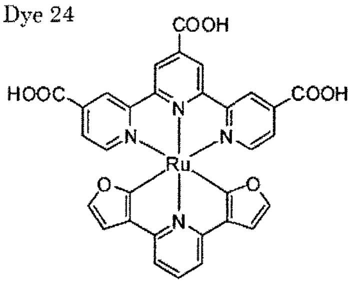

Dye 25

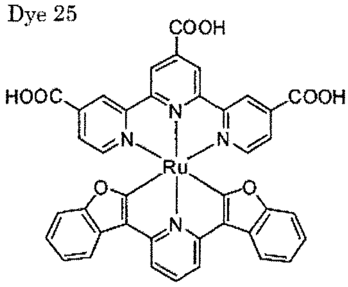

Dye 26

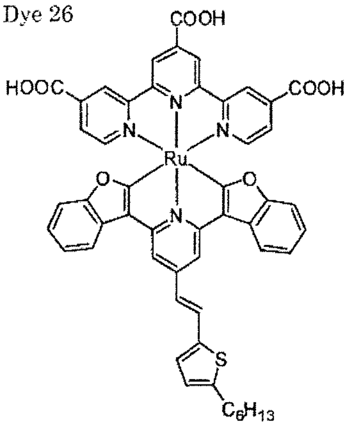

The metal complex dye according to the present invention can be easily synthesized using the ligands LD and LA according to the present invention and by methods described in US 2010/0258175 A1, JP-B-4298799, JP-A-2001-291534, or literatures cited in the official gazettes, Angew. Chem. Int. Ed., 2011, 50, 2054-2058, Chem. Commun., 2009, 5844-5846, reference literatures listed in these literatures, or in accordance with methods described in these patents or literatures.

The maximum absorption wavelength (λmax) in a solution (e.g. ethanol) of the metal complex dye of the present invention is preferably from 300 to 1,000 nm, more preferably from 350 to 950 nm, and particularly preferably from 370 to 900 nm.

«Photoelectric conversion element and dye-sensitized solar cell»

The photoelectric conversion element according to the present invention has a substrate, a transparent electrode, semiconductor fine particles, a metal complex dye having an anchoring group, an electrolyte and a counter electrode, and a member for insulating the transparent electrode from the counter electrode so as to hold this electrolyte. For example, as shown in Fig. 1, a photoelectric conversion element 10 has an electrically conductive support 1 formed

of a substrate and a transparent electrode, a photoconductor layer 2 sensitized by a dye 21 to be installed thereon, an electrolyte layer (electrolyte) 3 and a counter electrode 4. Here, in the present invention, a co-adsorbent 24 is preferably adsorbed on the above-described photoconductor layer together with the dye 21. The electrically conductive support 1 in which the photoconductor layer is installed functions as a working electrode in the photoelectric conversion element 10. In this embodiment, the photoelectric conversion element 10 is shown as a system 100, utilizing a dye-sensitized solar cell which enables the photoelectric conversion element 10 to use in a cell purpose which lets an operation means M to work with an external circuit 6.

In this embodiment, a light-receiving electrode (dye-adsorbed electrode) 5 is formed of an electrically conductive support 1; and a photoconductor layer 2 having semiconductor fine particles 22 on which the dye 21 to be coated thereon is adsorbed. In this embodiment, for convenience of illustration, the light-receiving electrode 5 is shown in a manner in which the electrolyte can be contained in the light-receiving electrode 5, but the electrolyte may be regarded not to be contained. The photoconductor layer 2 being the semiconductor layer is designed according to the intended purpose, and may have a monolayer constitution or a multilayer constitution. The dye 21 in a monolayered photoconductor layer may be constituted of one kind or a mixture of various kinds. Light incident to the photoconductor layer 2 being the semiconductor layer excites the dye 21. The excited dye has electrons having high energy, and these electrons are transferred from the dye 21 to a conduction band of the semiconductor fine particles 22, and further reach the electrically conductive support 1 by diffusion. At this time, the dye 21 is formed into an oxidant. The electrons on the electrode, while working in an external circuit 6, return through the counter electrode 4 to the photoconductor layer 2 in which the oxidant of the dye 21 exists to function as the solar cell.

The photoelectric conversion element according to this embodiment of the present invention includes, on an electrically conductive support, a photoconductor layer that has a layer containing porous semiconductor fine particles on which a metal complex dye described above has been adsorbed. A part of the dye at this moment may be dissociated into the electrolyte or so. The photoconductor layer is designed in accordance with the intended use, and may have a single layer structure or a multilayer structure. The photoconductor layer in the photoelectric conversion element according to this embodiment includes semiconductor fine particles on which the specific sensitizing dye has been adsorbed. Therefore, the photoconductor layer shows high sensitivity, and when used for the photoelectrochemical cell, high conversion efficiency and durability can be achieved.

It is noted that it is not particularly needed to specify the top and bottom of the photoelectric conversion element. In the present specification, however, when describing on the basis of the graphically-illustrated embodiment, the counter electrode 4 side is designated as the upside (top) direction, while the support 1 side that corresponds to the light-receiving side is designated as the underside (bottom) direction.

In the present invention, regarding materials for use in the photoelectric conversion element and the dye-sensitized solar cell, and a method of producing each member, ordinary ones in this kind may be adopted, and reference can be made to, for example, U.S. Patent No. 4,927,721, ditto, 4,684,537, ditto, 5,0843, 65, ditto, 5,350,644, ditto, 5,463,057, ditto, 5,525,440, JP-A-H7-249790, JP-A-2004-220974 or JP-A-2008-135197. An outline will be described on a main member below.

(Electrolyte composition)

Examples of the redox pair contained in the electrolyte composition for use in the photoelectric conversion element of the present invention include a combination of iodine and an iodide (for example, lithium iodide, tetrabutylammonium iodide, tetrapropylammonium iodide, or the like), a combination of an alkylviologen (for example, methylviologen chloride, hexylviologen bromide, or benzylviologen tetrafluoroborate) and a reductant thereof, a combination of a polyhydroxybenzene (for example, hydroquinone, naphthohydroquinone, or the like) and an oxidant thereof, a combination of a divalent iron complex and a trivalent iron complex (for example, a combination of potassium ferricyanide and potassium ferrocyanide), and a combination of a divalent cobalt complex and a trivalent cobalt complex. Among these, a combination of iodine and an iodide, and a combination of a divalent cobalt complex and a trivalent cobalt complex, are preferred.

- Cobalt Complex -

The cobalt complex is preferably a complex represented by formula (CC).

Co(LL)ma(X1)mb·CI    Formula (CC)

In formula (CC), LL represents a bidentate or terdentate ligand. X1 represents a monodentate ligand. ma represents an integer of 0 to 3. mb represents an integer of 0 to 6. CI represents a counter ion in the case where the counter ion is necessary to neutralize a charge in formula (CC).

X1 represents a monodentate ligand, and examples thereof includes: a monodentate ligand which coordinates by an anion selected from the group consisting of acyloxy anion, acylthio anion, thioacyloxy anion, thioacylthio anion, acylaminooxy anion, thiocarbamate anion, dithiocarbamate anion, thiocarbonate anion, dithiocarbonate anion, trithiocarbonate anion, acyl anion, thiocyanate anion, isothiocyanate anion, cyanate anion, isocyanate anion, cyano anion, alkylthio anion, arylthio anion, alkoxy anion, and aryloxy anion; or a monodentate ligand which coordinates by a group derived from these anions; or a monodentate ligand selected from the group of anions, atoms or compounds (including compounds in which a hydrogen atom is substituted with the anion) consisting of a halogen atom, cyano, carbonyl, dialkylketone, carbonamide, thiocarbonamide, and thiourea. In a case where the ligand X contains an alkyl group, an alkenyl group, an alkynyl group, an alkylene group or the like, these may be a straight chain or a branched chain, and these may be substituted or unsubstituted. Further, in a case where the ligand X contains an aryl group, a heterocyclic group, a cycloalkyl group or the like, these may be substituted or unsubstituted, and may be a single ring or a condensed ring.

Examples of CI include those of Y in formula (I).

LL is preferably a ligand represented by formula (LC).

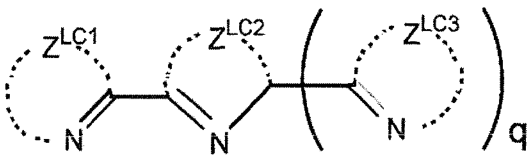

Formula (LC)

In formula (LC), $Z^{LC1}$, $Z^{LC2}$ and $Z^{LC3}$ each independently represent a group of atoms for forming a 5- or 6-membered ring. Each of $Z^{LC1}$, $Z^{LC2}$ and $Z^{LC3}$ may have a substituent, and may form a ring-closure together with an adjacent ring through a substituent. q represents 0 or 1. Examples of the substituent include the substituent T described below.

X1 is preferably a halogen ion.

The ligand represented by formula (LC) is preferably a ligand represented by any one of formulas (LC-1) to (LC-3).

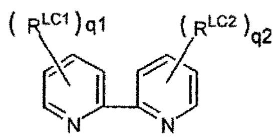

Formula (LC-1)

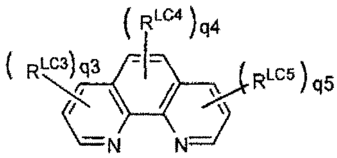

Formula (LC-2)

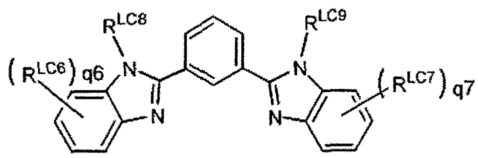

Formula (LC-3)

$R^{LC1}$ to $R^{LC9}$ each represent a substituent. q1, q2, q6 and q7 each independently represent an integer of 0 to 4. q3 and q5 each independently represent an integer of 0 to 3. q4 represents an integer of 0 to 2.

In formulas (LC-1) to (LC-3), examples of the substituent $R^{LC1}$ to $R^{LC9}$ include an aliphatic group, an aromatic group, a heterocyclic group or the like. Specific examples of the substituent include an alkyl group, an alkoxy group, an alkylthio group, an aryl group, an aryloxy group, an arylthio group, and a heterocyclic. Preferred examples include an alkyl group (for example, methyl, ethyl, n-butyl, n-hexyl, isobutyl, sec-butyl, t-butyl, n-dodecyl, cyclohexyl, or benzyl), a substituted-aryl group (for example, phenyl, tolyl, or naphthyl), and an alkoxy group (for example, methoxy, ethoxy, isopropoxy, or butoxy).

Specific examples of the cobalt complex represented by formula (LC) include the followings.

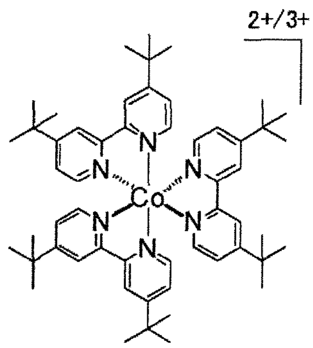

LL-1

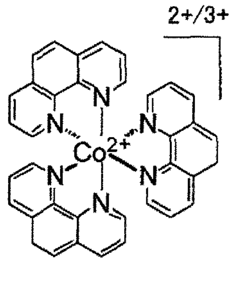

LL-2

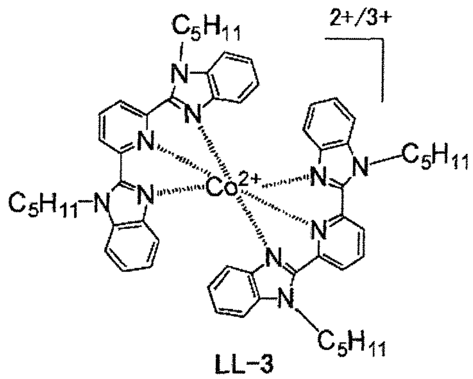

LL-3

When the compound represented by the formula (LC) is a cation of iodide salt, the cation of iodide salt preferably includes a nitrogen-containing aromatic cation of a 5-membered ring or 6-membered ring. Especially, in the case where the ligand represented by formula (LC) is not an iodide salt, the ligand is preferably used in combination with an iodide salt of pyridinium salts, imidazolium salts, triazolium salts or the like, as described in Japanese re-publication of WO95/18456, JP-A-8-259543, Denki Kagaku (Electrochemistry), Vol. 65, No. 11, page 923 (1997), and the like.

In the electrolyte composition used for the photoelectric conversion element according to the present invention, iodine is preferably contained together with a heterocyclic quaternary salt compound.

A content of iodine is preferably in the range of 0.1 to 20% by mass, and more preferably, in the range of 0.5 to 5% by mass, based on the total electrolyte composition.

- Co-adsorbent -

In the photoelectric conversion element of the present invention, a co-adsorbent is preferably used in combination with the metal complex dye of the present invention or another dye to be used if necessary. As such a co-adsorbent, a co-adsorbent having a carboxyl group or a salt thereof is preferable, and examples of the co-adsorbent include a fatty acid and a compound having a steroid skeleton. The fatty acid may be a saturated fatty acid or an unsaturated fatty acid. Examples thereof include a butanoic acid, a hexanoic acid, an octanoic acid, a decanoic acid, a hexadecanoic acid, a dodecanoic acid, a palmitic acid, a stearic acid, an oleic acid, a linoleic acid, and a linolenic acid.

Examples of the compound having a steroid skeleton include a cholic acid, a glycocholic acid, a chenodeoxycholic acid, a hyocholic acid, a deoxycholic acid, a lithocholic acid, and ursodeoxycholic acid. Among these, a cholic acid, a deoxycholic acid, and a chenodeoxycholic acid are preferable; and a chenodeoxycholic acid is more preferable.

A preferred co-adsorbent is a compound represented by formula (CA).

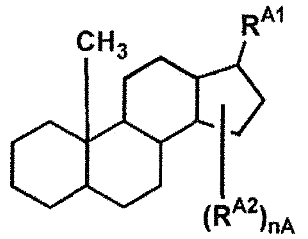

Formula (CA)

In formula (CA), $R^{A1}$ represents a substituent having an acidic group. $R^{A2}$ represents a substituent. nA represents an integer of 0 or more.

The acidic group has the same meaning as described above. Moreover, specific examples of the substituent in $R^{A1}$ or $R^{A2}$ include the substituent T described below.

nA is preferably from 2 to 4.

Examples of the specific compounds include a compound that is exemplified as the compound having a steroid skeleton.

By adsorbing on the semiconductor fine-particles, the co-adsorbent that can be used in the present invention exhibits an effect on suppressing the inefficient association of the dye, and preventing reverse electron transfer from the oxide semiconductor fine-particle surface to the redox system in the electrolyte. An amount to be used of the co-adsorbent is not particularly limited, and it is preferred, from the viewpoint of exhibiting effectively the effects, that the amount is preferably from 1 to 200 moles, more preferably from 10 to 150 moles, and particularly preferably from 20 to

50 moles, with respect to 1 mole of a total of dyes to be used including the metal complex dye of the present invention.

<Substituent T>

The specification uses an expression "compound" (including complex and dye) to mean, in addition to the compound itself, its salts, its complex and its ion. Further, a substituent with which substitution or non-substitution is not explicitly described in the present specification (the same applies to a linking group and a ligand), means that the substituent may have an arbitrary substituent. The same is also true on a compound with which substitution or non-substitution is not explicitly described. Preferable examples of the substituent include the following substituent T.

In the present specification, the simple description only as a "substituent" means to refer to this substituent T. Further, in a case where each of the substituents, for example, like an alkyl group, is described in a simplistic form, both a preferable range and specific examples for the corresponding group of the substituent T are applied to.

The substituent T includes the followings:

an alkyl group (preferably an alkyl group having 1 to 20 carbon atoms, e.g. methyl, ethyl, isopropyl, t-butyl, pentyl, heptyl, 1-ethylpentyl, benzyl, 2-ethoxyethyl, 1-carboxymethyl, or trifluoromethyl), an alkenyl group (preferably an alkenyl group having 2 to 20 carbon atoms, e.g. vinyl, allyl, or oleyl), an alkynyl group (preferably an alkynyl group having 2 to 20 carbon atoms, e.g. ethynyl, butadiynyl, or phenylethynyl), a cycloalkyl group (preferably a cycloalkyl group having 3 to 20 carbon atoms, e.g. cyclopropyl, cyclopentyl, cyclohexyl, or 4-methylcyclohexyl), an cycloalkenyl group (preferably a cycloalkenyl group having 5 to 20 carbon atoms, e.g. cyclopentenyl, or cyclohexenyl), an aryl group (preferably an aryl group having 6 to 26 carbon atoms, e.g. phenyl, 1-naphthyl, 4-methoxyphenyl, 2-chlorophenyl, or 3-methylphenyl), a heterocyclic group (preferably a 5- or 6-membered heterocyclic group having 2 to 20 carbon atoms and at least one oxygen atom, sulfur atom, or nitrogen atom, e.g. 2-pyridyl, 4-pyridyl, 2-imidazolyl, 2-benzimidazolyl, 2-thiazolyl, or 2-oxazolyl), an alkoxy group (preferably an alkoxy group having 1 to 20 carbon atoms, e.g. methoxy, ethoxy, isopropyloxy, or benzyloxy), an alkenyloxy group (preferably an alkenyloxy group having 2 to 20 carbon atoms, e.g. vinyloxy or allyloxy), an alkynyloxy group (preferably an alkynyloxy group having 2 to 20 carbon atoms, e.g. 2-propenyloxy or 4-butynyloxy), an aryloxy group (preferably an aryloxy group having 6 to 26 carbon atoms, e.g. phenoxy,1-naphthyloxy, 3-methylphenoxy, or 4-methoxyphenoxy), a heterocyclic oxy group (e.g. imidazolyloxy, benzoimidazolyloxy, thiazolyloxy, benzothiazolyloxy, triazinyloxy, or purinyloxy); an alkoxycarbonyl group (preferably an alkoxycarbonyl group having 2 to 20 carbon atoms, e.g. ethoxycarbonyl, or 2-ethylhexyloxycarbonyl), an aryloxycarbonyl group (preferably an aryloxycarbonyl group having 6 to 20 carbon atoms, e.g. phenyloxycarbonyl, or naphthyloxycarbonyl), an amino group (preferably an amino group having 0 to 20 carbon atoms including an alkylamino group, an alkenylamino group, an alkynylamino group, a cycloalkylamino group, a cycloalkenylamino group, an arylamino group, and a heterocyclic amino group, e.g. amino, N,N-dimethylamino, N,N-diethylamino, N-ethylamino, N-allylamino, N-(2-propinyl)amino, N-cyclohexylamino, N-cyclohexenylamino, anilino, pyridylamino, imidazolylamino, benzimidazolylamino, thiazolylamino, benzothiazolylamino, or triazinylamino), a sulfamoyl group (preferably a sulfamoyl group having 0 to 20 carbon atoms, preferably an alkyl- or aryl-sulfamoyl group, e.g. N,N-dimethylsulfamoyl, or N-phenylsulfamoyl), an acyloxy group (preferably an acyloxy group having 1 to 20 carbon atoms, e.g. acetyloxy, or benzoyloxy), a carbamoyl group (preferably a carbamoyl group having 1 to 20 carbon atoms, preferably an alkyl- or aryl-carbamoyl group, e.g. N,N-dimethylcarbamoyl or N-phenylcarbamoyl), an acylamino group (preferably an acylamino group having 1 to 20 carbon atoms, e.g. acetylamino or benzoylamino), a sulfonamide group (preferably a sulfonamide group having 0 to 20 carbon atoms, preferably an alkyl- or aryl-sulfonamide group, e.g. methane sulfonamide, benzene sulfonamide, N-methyl methane sulfonamide, or N-ethyl benzene sulfonamide), an alkylthio group (preferably an alkylthio group having 1 to 20 carbon atoms, e.g. methylthio, ethylthio, isopropylthio, or benzylthio), an arylthio group (preferably an arylthio group having 6 to 26 carbon atoms, e.g. phenylthio, 1-naphthylthio, 3-methylphenylthio, or 4-methoxyphenylthio), an alkyl- or aryl-sulfonyl group (preferably a sulfonyl group having 1 to 20 carbon atoms, e.g. methylsulfonyl, ethylsulfonyl, or benzene sulfonyl), a silyl group (preferably a silyl group having 1 to 20 carbon atoms, preferably an alkyl-, aryl-, alkoxy-, or aryloxy-substituted silyl group, e.g. triethylsilyl, triphenylsilyl, diethylbenzylsilyl, or dimethylphenylsilyl), a silyloxy group (preferably a silyloxy group having 1 to 20 carbon atoms, preferably an alkyl-, aryl-, alkoxy-, or aryloxy-substituted silyloxy group, e.g. triethylsilyloxy, triphenylsilyloxy, diethylbenzylsilyloxy, or dimethylphenylsilyloxy), a hydroxyl group, a cyano group, a nitro group, a halogen atom (e.g. fluorine, chlorine, bromine, or iodine atom), a carboxyl group, a sulfo group, a phosphonyl group, a phosphoryl group, and a boric-acid group; more preferably an alkyl group, an alkenyl group, an aryl group, a heterocyclic group, an alkoxy group, an aryloxy group, an alkoxycarbonyl group, an amino group, an acylamino group, a cyano group, or a halogen atom; and particularly preferably an alkyl group, an alkenyl group, a heterocyclic group, an alkoxy group, an alkoxycarbonyl group, an amino group, an acylamino group, or a cyano group.

When the compound or the substituent or the like contains an alkyl group or an alkenyl group, these may be a straight chain or a branched chain, and these may be substituted or unsubstituted. Further, in the case of containing an aryl group, a heterocyclic group or the like, these may be a single ring or a condensed ring, and may be substituted or unsubstituted.

In the present invention, regarding materials for use in the photoelectric conversion element and the dye-sensitized solar cell, and a method of producing each member, ordinary ones in this kind may be adopted, and reference can be made to, for example, U.S. Patent No. 4,927,721, ditto, 4,684,537, ditto, 5,0843, 65, ditto, 5,350,644, ditto, 5,463,057, ditto, 5,525,440, JP-A-7-249790, JP-A-2004-220974, and JP-A-2008-135197. An outline will be described on a main member below.

The electrically-conductive support is preferably a support having electroconductivity per se, such as a metal, or a support of glass or plastic having an electrically-conductive layer on the surface. In addition to the glass and plastic, ceramic (JP-A-2005-135902), an electrically-conductive resin (JP-A-2001-160425) or the like may be used as the support. The support may be provided with a light management function at the surface, and for example, the anti-reflective film having a high refractive index film and a low refractive index oxide film alternately laminated as described in JP-A-2003-123859, and the light guide function as described in JP-A-2002-260746 may be mentioned.

The thickness of the electrically-conductive layer is preferably 0.01 to 30 μm, more preferably 0.03 to 25 μm, and particularly preferably 0.05 to 20 μm.

It is preferable that the electrically-conductive support is substantially transparent. The term "substantially transparent" means that the transmittance of light is 10% or more, preferably 50% or more, particularly preferably 80% or more. As the transparent electrically-conductive support, a support formed from glass or plastic and coated with an electrically-conductive metal oxide is preferable. In this case, the amount of coating of the electrically-conductive metal oxide is preferably 0.1 to 100 g, per square meter of the support made of glass or plastic. In the case of using a transparent electrically-conductive support, it is preferable that light is incident from the support side.

Regarding the semiconductor fine-particles, fine-particles of chalcogenides of metals (for example, oxides, sulfides and selenides), or fine-particles of perovskites may be used with preference. Preferred examples of the chalcogenides of metals include oxides of titanium, tin, zinc, tungsten, zirconium, hafnium, strontium, indium, cerium, yttrium, lanthanum, vanadium, niobium or tantalum, cadmium sulfide, and cadmium selenide. Preferred examples of the perovskites include strontium titanate, and calcium titanate. Among these, titanium oxide, zinc oxide, tin oxide, and tungsten oxide are particularly preferred.

Examples of the crystal structure of titania include structures of anatase type, brookite type and rutile type, and anatase type and brookite type structures are preferred. A titania nanotube/nanowire/nanorod may be mixed with titania fine-particles or may be used as a semiconductor electrode.

A particle size of the semiconductor fine-particles is expressed in terms of an average particle size using a diameter when a projected area is converted into a circle, and is preferably 0.001 to 1 μm as primary particles, and 0.01 to 100 μm as an average particle size of dispersions. Examples of the method for coating the semiconductor fine-particles on the electrically-conductive support include a wet method, a dry method or other methods.

It is preferable to form a short circuit-preventing layer between the transparent electrically-conductive film and the photoconductor layer ("semiconductor layer" or "oxide semiconductor layer"), so as to prevent reverse current due to a direct contact between the electrolyte liquid and the electrode. It is preferable to employ a spacer or a separator, so as to prevent contact between the photoelectrode and the counter electrode. It is preferable for the semiconductor fine-particles to have a large surface area, so that a large amount of dye can adsorb to the surface. For example, while the semiconductor fine-particles have been coated on the support, the surface area is preferably 10 times or more, and more preferably 100 times or more, relative to the projected surface area. The upper limit of this value is not particularly limited, and the upper limit is generally about 5,000 times. In general, as the thickness of the semiconductor fine-particle layer increases, the amount of dye that can be supported per unit area increases, and therefore, the light absorption efficiency is increased. However, since the diffusion distance of generated electrons increases along, the loss due to charge recombination is also increased. Although a preferred thickness of the photoconductor layer containing semiconductor fine-particles may vary with the utility of the element, the thickness is typically 0.1 to 100 μm. In the case of using the photoelectric conversion element for a dye-sensitized solar cell, the thickness of the semiconductor fine-particle layer is preferably 1 to 50 μm, and more preferably 3 to 30 μm. The semiconductor fine-particles may be calcined after being applied on the support, at a temperature of 100 to 800°C for 10 minutes to 10 hours, so as to bring about cohesion of the particles. When a glass support is used, the film-forming temperature is preferably 400 to 600°C.

The amount of coating of the semiconductor fine-particles per square meter of the support is preferably 0.5 to 500 g, and more preferably 5 to 100 g. The overall amount of use of the dye is preferably 0.01 to 100 millimoles, more preferably 0.1 to 50 millimoles, and particularly preferably 0.1 to 10 millimoles, per square meter of the support. In this case, the amount of use of the metal complex dye of the present invention is preferably set to 5% by mole or more. The amount of the dye adsorbed to the semiconductor fine-particles is preferably 0.001 to 1 millimole, and more preferably 0.1 to 0.5 millimoles, based on 1 g of the semiconductor fine-particles. When the amount of the dye is set to such a

range, the sensitization effect can be sufficiently obtained. To the contrary, a too small amount of the dye causes an insufficient sensitization effect, and an excessively large amount of the dye causes floatation of a dye not deposited on the semiconductor to reduce the sensitization effect.

When the dye is a salt, a counter ion of the specific metal complex dye is not particularly limited. Examples thereof include an alkali metal ion and a quaternary ammonium ion.

After the dye has been adsorbed, the surface of the semiconductor fine-particles may be treated using amines. Preferred examples of the amines include pyridines (e.g., 4-tert-butylpyridine, and polyvinylpyridine). These may be used directly when the compounds are liquids, or may be used in a state of being dissolved in an organic solvent. The charge transfer layer is a layer having a function to replenish electrons to the oxide form of the dye, and it is provided between the light-receiving electrode and the counter electrode (an opposite electrode). Representative examples thereof include a liquid electrolyte having a redox pair dissolved in an organic solvent, a so-called gel electrolyte in which a liquid having a redox pair dissolved in an organic solvent is impregnated in a polymer matrix, and a molten salt containing a redox pair.

A solid-state charge-transport system, such as a p-type semiconductor or a hole-transporting material, may also be used in place of a liquid electrolyte and a quasi-solid-state electrolyte as described above. For a solid charge-transport layer, an organic hole-transporting material may be used.

The redox pair serves as an electron carrier, and therefore needs a certain degree of concentration. A preferable concentration thereof in total is 0.01 mole/l or more, more preferably 0.1 mole/l or more, and particularly preferably 0.3 mole/l or more. The upper limit of this is not particularly limited, and generally about 5 mole/l.

The counter electrode is preferably an electrode working as a positive electrode in the dye-sensitized solar cell (photoelectrochemical cell). The counter electrode usually has the same meaning as the electrically-conductive support described above, but in a construction which is likely to maintain a sufficient strength, a support is not necessarily required. A preferred structure of the counter electrode is a structure having a high charge collecting effect. At least one of the electrically-conductive support and the counter electrode as mentioned above should be substantially transparent, in order for light to reach the photoconductor layer. In the dye-sensitized solar cell of the present invention, the electrically-conductive support is preferably transparent to allow sunlight to inject from the support side. In this case, the counter electrode has further preferably properties of reflecting light. As the counter electrode of the dye-sensitized solar cell, a glass or plastic plate on which a metal or an electrically-conductive oxide is deposited is preferable, and a glass plate on which platinum is deposited is particularly preferable. In the dye-sensitized solar cell, a lateral side of the cell is preferably sealed with a polymer, an adhesive, or the like, in order to prevent evaporation of the component. Characteristics of the thus obtained dye-sensitized solar cell according to the present invention include, preferably, upon 100 $mW/cm^2$ at AM 1.5 G, an open-circuit voltage (Voc) of 0.01 to 1.5 V, a short-circuit current density (Jsc) of 0.001 to 20 $mA/cm^2$, a fill factor (FF: fill factor or form factor) of 0.1 to 0.9 and a photoelectric conversion efficiency ($\eta$) of 0.001 to 25%.

«Dye adsorption liquid composition, semiconductor electrode using the same, and production method of dye-sensitized solar cell»

In the present invention, the dye-adsorbed electrode is preferably produced using a dye adsorption liquid composition containing the metal complex dye of the present invention.

In the foregoing dye adsorption liquid composition, the metal complex dye of the present invention is dissolved in a solvent, and a co-adsorbent and other ingredients may be contained therein, as needed.

The foregoing solvent includes solvents described in JP-A 2001-291534, but the solvent is not particularly limited thereto. In the present invention, organic solvents are preferred. More preferred are alcohols, amides, nitriles, hydrocarbons, and a mixed solvent of two or more kinds of these solvents. As a mixed solvent, preferred are those of alcohols and a solvent selected from amides, nitriles, or hydrocarbons. More preferred are mixed solvents of alcohols and amides and mixed solvents of alcohols and hydrocarbons, and particularly preferred are mixed solvents of alcohols and amides.

The dye adsorption liquid composition preferably contains a co-adsorbent, and the co-adsorbent is preferably the foregoing ones. Among them, the compound represented by formula (CA) is preferred.

Preferred is the dye adsorption liquid composition of the present invention in which a concentration of the metal complex dye and the co-adsorbent have been adjusted so that the dye adsorption liquid composition can be used as it is when a photoelectric conversion element or a dye-sensitized solar cell is prepared. In the present invention, the metal complex dye of the present invention is preferably contained in an amount of from 0.001 to 0.1 % by mass.

In the dye adsorption liquid composition, adjustment of water content is preferred in particular, and thus in the present invention, it is preferred that the content (content rate) of water is adjusted to the range of from 0 to 0.1% by mass.

Similarly, adjustment of water content of the electrolytic solution in a photoelectric conversion element and a dye-sensitized solar cell is preferred, in order to achieve effectively the effects of the present invention. Thus, it is preferred that the content (content rate) of water in the electrolytic solution is adjusted to the range of from 0 to 0.1% by mass.

The foregoing adjustment of the electrolytic solution is preferably carried out with the dye adsorption liquid composition in particular.

In the present invention, preferred is a semiconductor electrode for dye-sensitized solar cell in which the metal complex dye derived from the use of the dye adsorption liquid composition has been carried on a semiconductor surface provided on the semiconductor electrode.

Further, it is preferred to produce a dye-sensitized solar cell, using the dye adsorption liquid composition, thereby having a metal complex dye carried on the semiconductor fine-particle surface provided on the semiconductor electrode.

The present invention can be applied to the photoelectric conversion elements and the dye-sensitized solar cells described, for example, in Japanese Patent No. 4260494, JP-A-2004-146425, JP-A-2000-340269, JP-A-2002-289274, JP-A-2004-152613, JP-A-9-27352. In addition, the present invention can be applied to the photoelectric conversion elements and the dye-sensitized solar cells described, for example, in JP-A-2004-152613, JP-A-2000-90989, JP-A-2003-217688, JP-A-2002-367686, JP-A-2003-323818, JP-A-2001-43907, JP-A-2000-340269, JP-A-2005-85500, JP-A-2004-273272, JP-A-2000-323190, JP-A-2000-228234, JP-A-2001-266963, JP-A-2001-185244, JP-T-2001-525108 (the term "JP-T" means a published Japanese translation of a PCT patent application), JP-A-2001-203377, JP-A-2000-100483, JP-A-2001-210390, JP-A-2002-280587, JP-A-2001-273937, JP-A-2000-285977, JP-A-2001-320068.

## EXAMPLES

The present invention will be described in more detail based on examples given below, but the invention is not meant to be limited by these.

<Example of synthesis of dye>

[Synthesis of exemplified dye 1]

A compound LD-1-1 was synthesized according to the method of the following scheme to synthesize an exemplified dye 1.

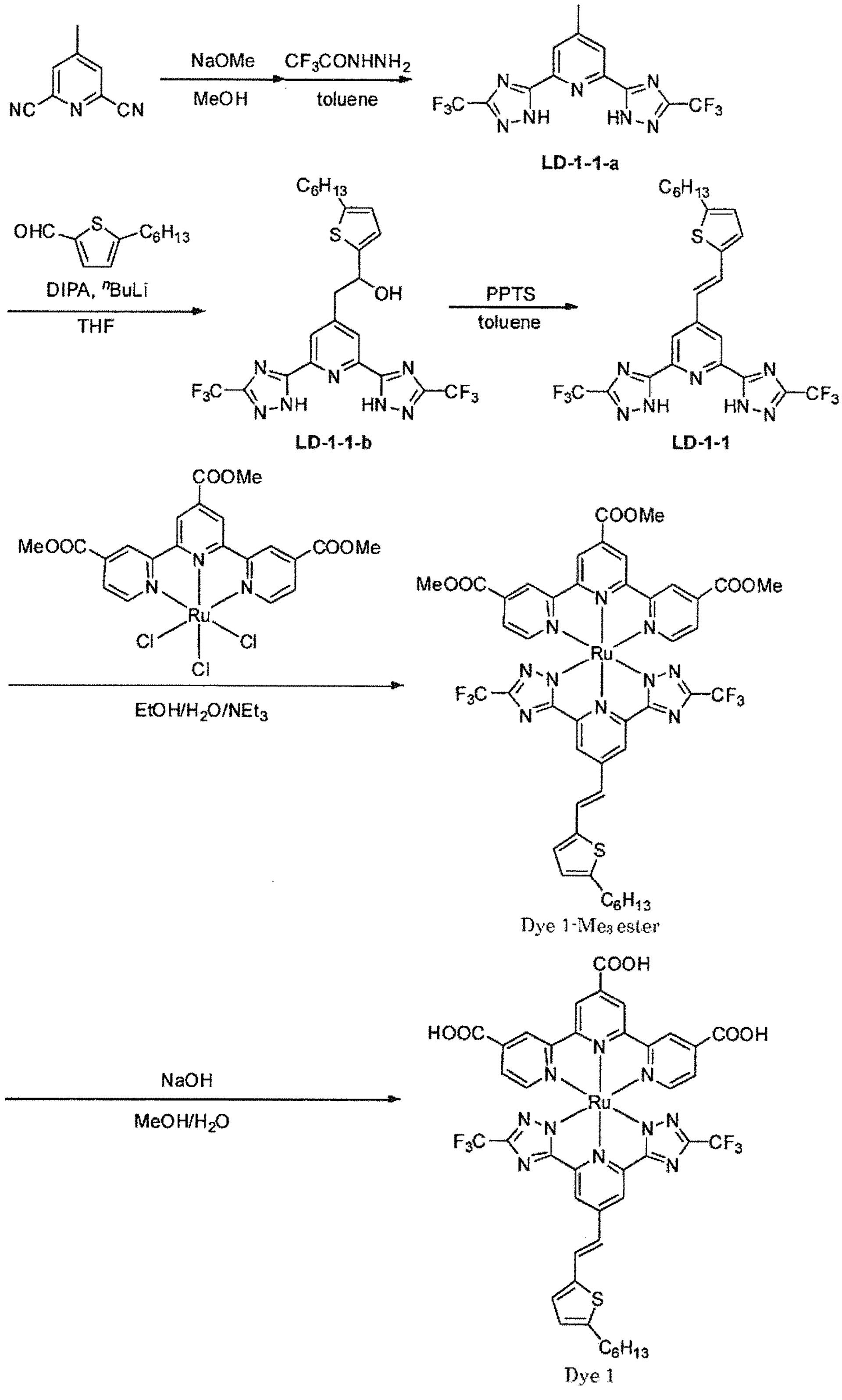


(1) Synthesis of compound LD-1-1-a

Under a nitrogen atmosphere, 7.0 g of 2,6-dicyano-4-methylpyridine was added to 30 mL of methanol, and the resultant mixture was stirred. Then, 19.5 mL of 28% methanol solution of sodium methoxide was added thereto, and the resultant mixture was stirred at room temperature for 1 hour. Then, 5.5 mL of acetic acid was added dropwise to this solution, and the resultant mixture was stirred for 5 minutes. Then, 17 g of trifluoroacetohydrazide was added thereto,

and the resultant mixture was stirred at room temperature for 1 hour. Crystals gradually precipitated, and the crystals were separated by filtration, and collected. To the resulting crystals, 50 mL of toluene was added, and the resultant mixture was heated and refluxed for 3 hours. After being cooled to room temperature, a crude product obtained by concentration under reduced pressure was purified by silica gel column chromatography to obtain 6.4 g of object.

(2) Synthesis of compound LD-1-1-b

Under a nitrogen atmosphere, 5.4 g of diisopropylamine was dissolved into 25 mL of tetrahydrofuran, and the resultant mixture was cooled to -20°C. Then, 35 mL of n-butyllithium (1.6 mol/L hexane solution) was added dropwise thereto, and the resultant mixture was stirred at -20°C for 30 minutes. Then, 50 mL of tetrahydrofuran solution of 6.4 g of compound LD-1-1-a was added dropwise thereto, and the resultant mixture was stirred at 0°C for 75 minutes. To this suspension, 10 mL of tetrahydrofuran solution of 3.5 g of 5-hexylthiophene-2-carboxyaldehyde was added dropwise, and the resultant mixture was stirred at room temperature for 90 minutes. Then, 100 mL of saturated aqueous solution of ammonium chloride was added thereto, and then subjected to liquid separation, and the resultant organic layer was condensed under reduced pressure. The resulting crude product was purified by silica gel column chromatography to obtain 8.4 g of object.

(3) Synthesis of compound LD-1-1

Under a nitrogen atmosphere, 8.4 g of compound LD-1-1-b and 3.7 g of PPTS (pyridinium p-toluenesulfonate) were added to 50 mL of toluene, and the resultant mixture was heated and refluxed for 4 hours. Liquid separation was performed using 50 mL of saturated aqueous solution of sodium hydrogencarbonate and 100 mL of ethyl acetate, and the resultant organic layer was condensed. The resulting crude product was recrystallized by methanol to obtain 6.6 g of object.

(4) Synthesis of dye 1-$Me_3$ ester

Under a nitrogen atmosphere, 615 mg of ($Me_3$-tctpy)$RuCl_3$ ($Me_3$-tctpy = 4,4',4"-tri(methoxycarbonyl)-2,2':6',2"-terpyridyl), 542 mg of compound LD-1-1 and 1.5 mL of triethylamine were added to a mixed solution of 500 mL of ethanol and 100 mL of water, and the resultant mixture was heated and refluxed for 3 hours. The resultant reaction solution was concentrated under reduced pressure, 20 mL of ethyl acetate and 200 mL of water were added thereto, the resultant mixture was subjected to liquid separation, and the resultant organic layer was condensed under reduced pressure. The resulting crude product was purified by silica gel column chromatography to obtain 626 mg of object.

(5) Synthesis of exemplified dye 1

Under a nitrogen atmosphere, 100 mg of dye 1-Me3 ester was added to 30 mL of methanol, and completely dissolved thereinto. Then, 10 mL of 1 N aqueous solution of sodium hydroxide was added thereto, and the resultant mixture was stirred at room temperature for 12 hours. The resultant reaction mixture was concentrated under reduced pressure, the resulting solid was dissolved into 30 mL of water, and 10 mL of 1 N aqueous solution of trifluoromethanesulfonic acid was added to this solution. A precipitated solid was collected by filtration, and washed with 10 mL of water, and subsequently with 10 mL of diethyl ether to obtain 92 mg of object.

[Synthesis of exemplified dye 2]

A ligand LD-1-25 was synthesized according to the method of the following scheme to synthesize a dye 2 in a manner similar to synthesis of the exemplified dye 1.

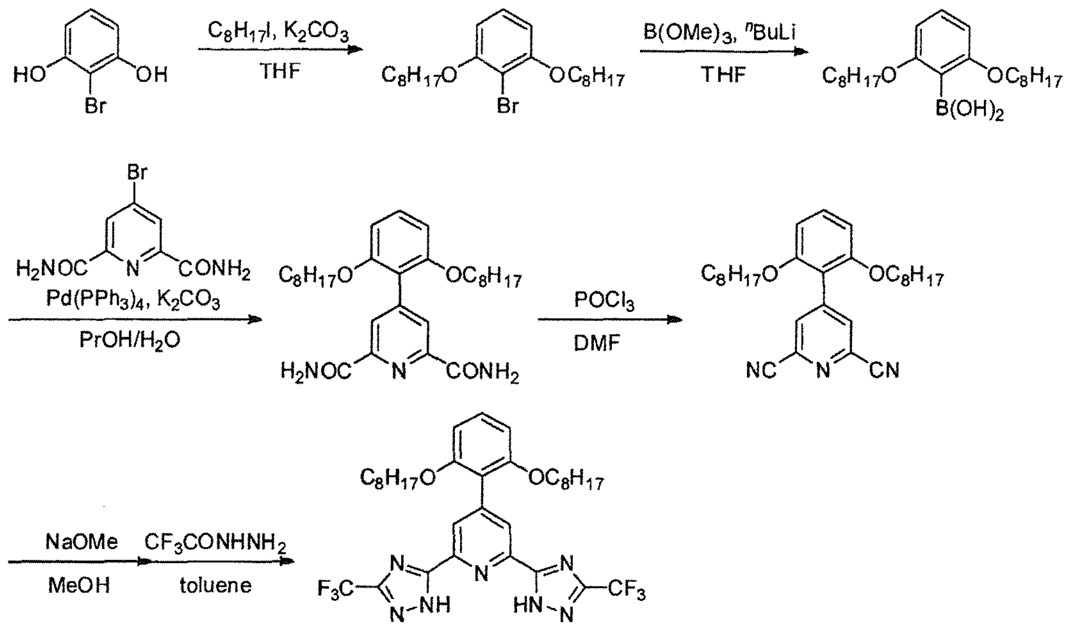


**LD-1-25**

[Synthesis of exemplified dye 3]

A compound LD-2-5-a was synthesized according to the method of the following scheme to synthesize an exemplified dye 3 by a method in accordance with the method for synthesizing the exemplified dye 1.

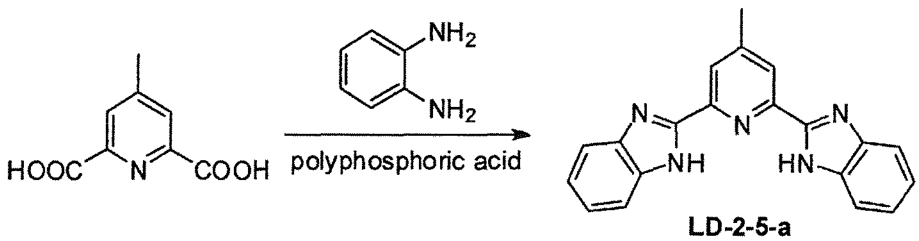


**LD-2-5-a**

[Synthesis of exemplified dye 4]

A compound LD-2-8-a was synthesized according to the method of the following scheme to synthesize an exemplified dye 4 by a method in accordance with the method for synthesizing the exemplified dye 1.

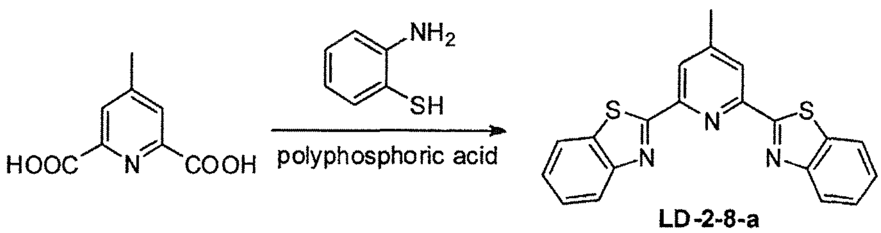


**LD-2-8-a**

[Synthesis of exemplified dye 5]

A ligand LD-3-1 was synthesized according to the method of the following scheme to synthesize an exemplified dye 5 by a method in accordance with the method for synthesizing the exemplified dye 1.

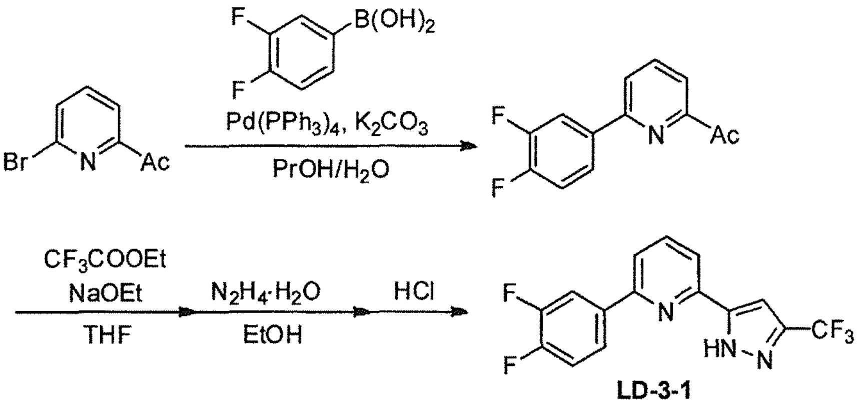


LD-3-1

[Synthesis of exemplified dye 6]

A compound LD-4-1-a was synthesized according to the method of the following scheme to synthesize an exemplified dye 6 by a method in accordance with the method for synthesizing the exemplified dye 1.

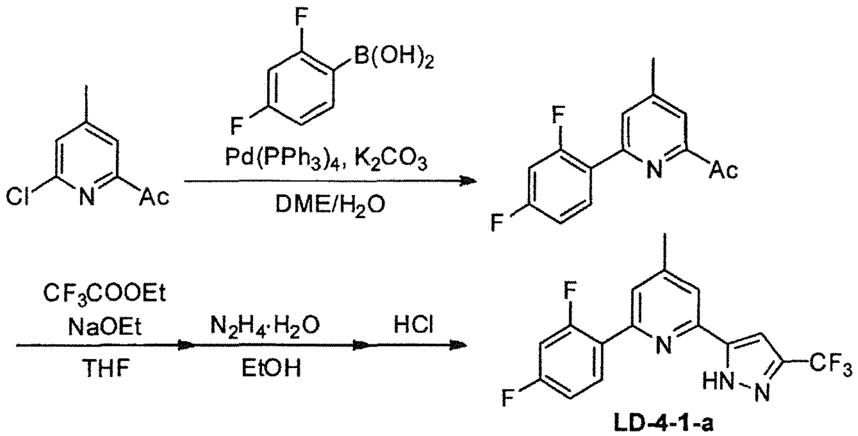


LD-4-1-a

[Synthesis of exemplified dye 7]

An LD-4-4-a was synthesized by a method in accordance with the method for synthesizing the ligand LD-3-1, and a ligand LD-4-4 was synthesized using this product according to the method of the following scheme. An exemplified dye 7 was synthesized by a method in accordance with the method for synthesizing the exemplified dye 1.

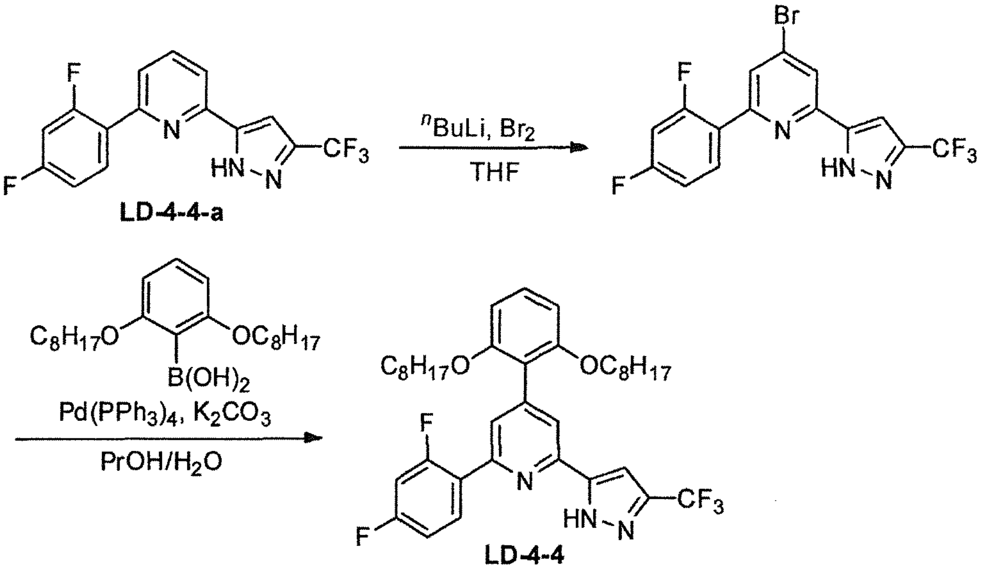


LD-4-4-a

LD-4-4

[Synthesis of exemplified dye 8]

A compound LD-3-11-a was synthesized according to the method of the following scheme to synthesize an exemplified dye 8 by a method in accordance with the method for synthesizing the exemplified dye 5.

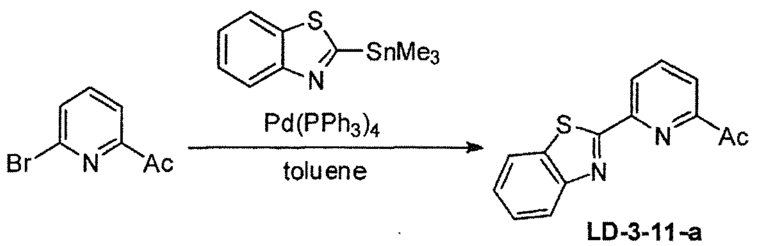


LD-3-11-a

[Synthesis of exemplified dye 9]

A ligand LA-2-6 was synthesized according to the method of the following scheme to synthesize an exemplified dye 9 by a method in accordance with the method for synthesizing the exemplified dye 2.

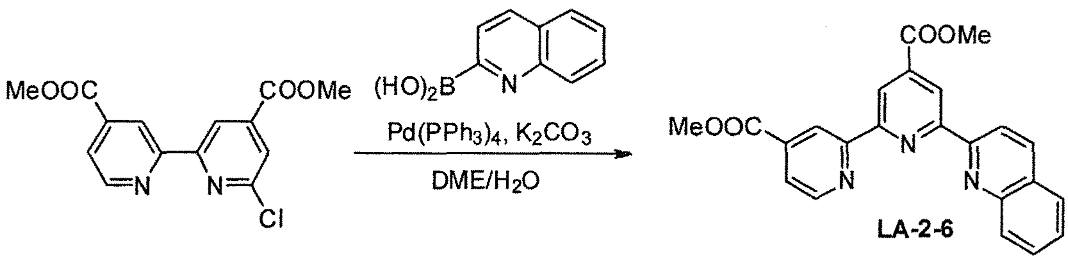


LA-2-6

[Synthesis of exemplified dye 10]

A ligand LA-2-1 was synthesized according to the method of the following scheme to synthesize an exemplified dye 10 by a method in accordance with the method for synthesizing the exemplified dye 4.

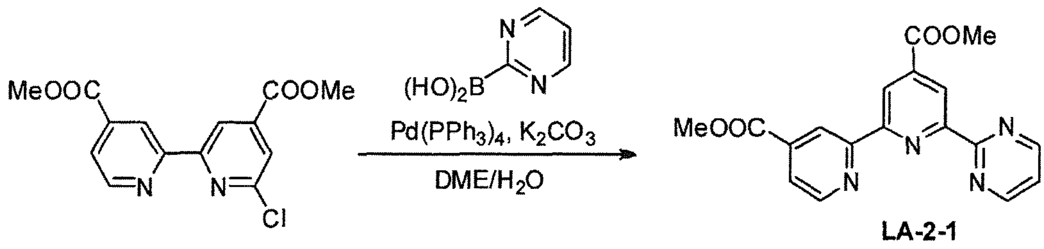

LA-2-1

[Synthesis of exemplified dye 11]

A ligand LA-2-13 was synthesized according to the method of the following scheme to synthesize an exemplified dye 11 by a method in accordance with the method for synthesizing the exemplified dye 5.

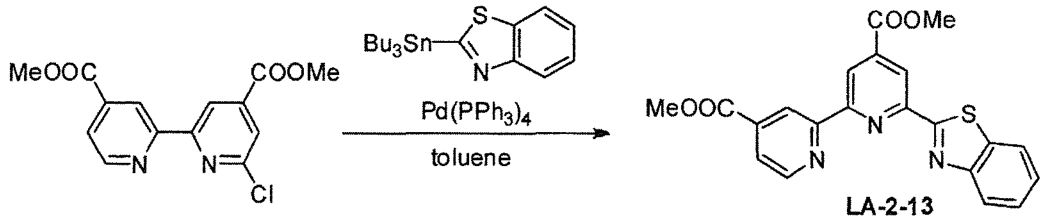

LA-2-13

[Synthesis of exemplified dye 12]

An exemplified dye 12 was synthesized using the ligand LA-2-13 synthesized by the above-described method and by a method in accordance with the method for synthesizing the exemplified dye 7.

[Synthesis of exemplified dye 13]

A ligand LA-3-6 was synthesized according to the method of the following scheme to synthesize an exemplified dye 13 by a method in accordance with the method for synthesizing the exemplified dye 1.

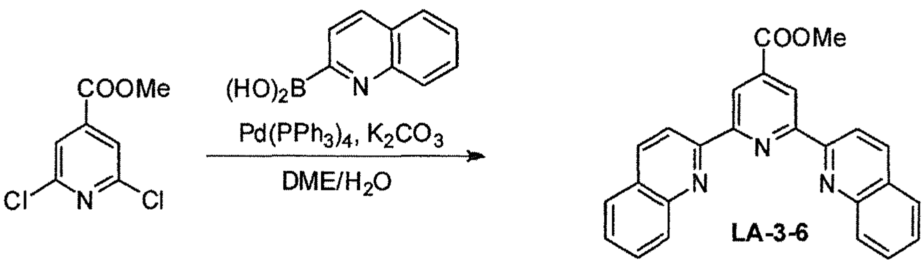

LA-3-6

[Synthesis of exemplified dye 14]

A ligand LA-3-8 was synthesized according to the method of the following scheme to synthesize an exemplified dye 14 by a method in accordance with the method for synthesizing the exemplified dye 5.

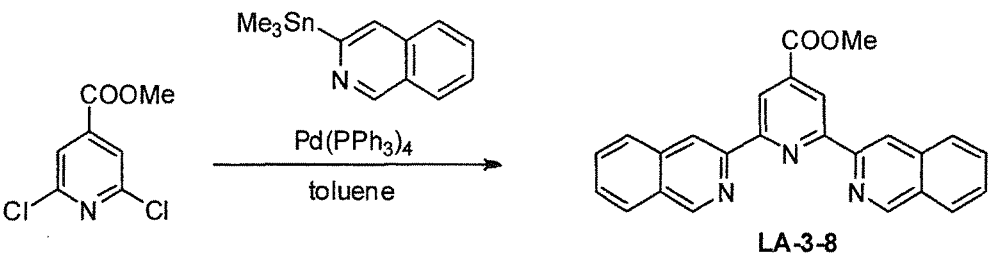

LA-3-8

[Synthesis of exemplified dye 15]

An exemplified dye 12 was synthesized using the ligand LA-2-13 synthesized by the above-described method and by a method in accordance with the method for synthesizing the exemplified dye 7.

[Synthesis of exemplified dye 16]

A ligand LA-3-11 was synthesized according to the method of the following scheme to synthesize an exemplified dye 16 by a method in accordance with the method for synthesizing the exemplified dye 5.

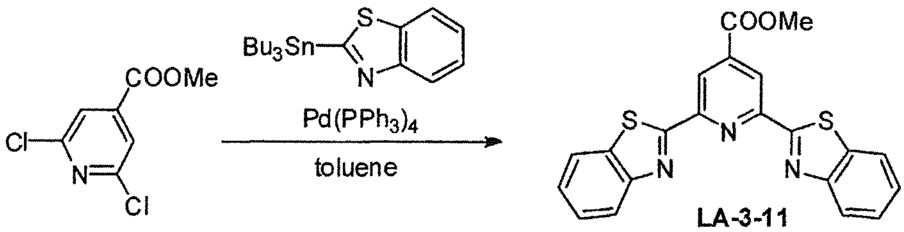


LA-3-11

[Synthesis of exemplified dyes 17 to 20]

A ligand LA-5-1, LA-5-3 or LA-5-4 was synthesized using commercially available boronic acid according to the following scheme.

In addition, the following Ar represents a 4-$C_6H_{13}$-phenyl group for the LA-5-1, a 5-$C_6H_{13}$-thienyl group for the LA-5-3, and a 4-(5-$C_6H_{13}$- thienyl) phenyl group for the LA-5-4.

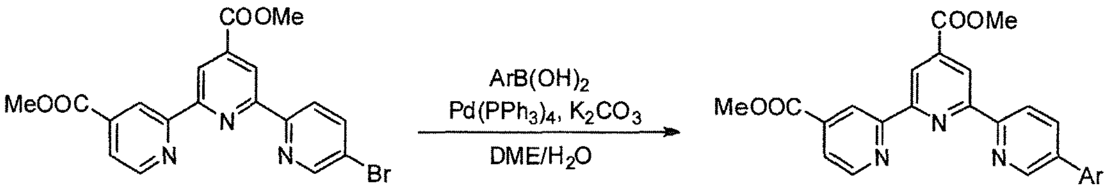


LA-5-1
LA-5-3
LA-5-4

A ligand LD-4-7 was synthesized according to the method of the following scheme.

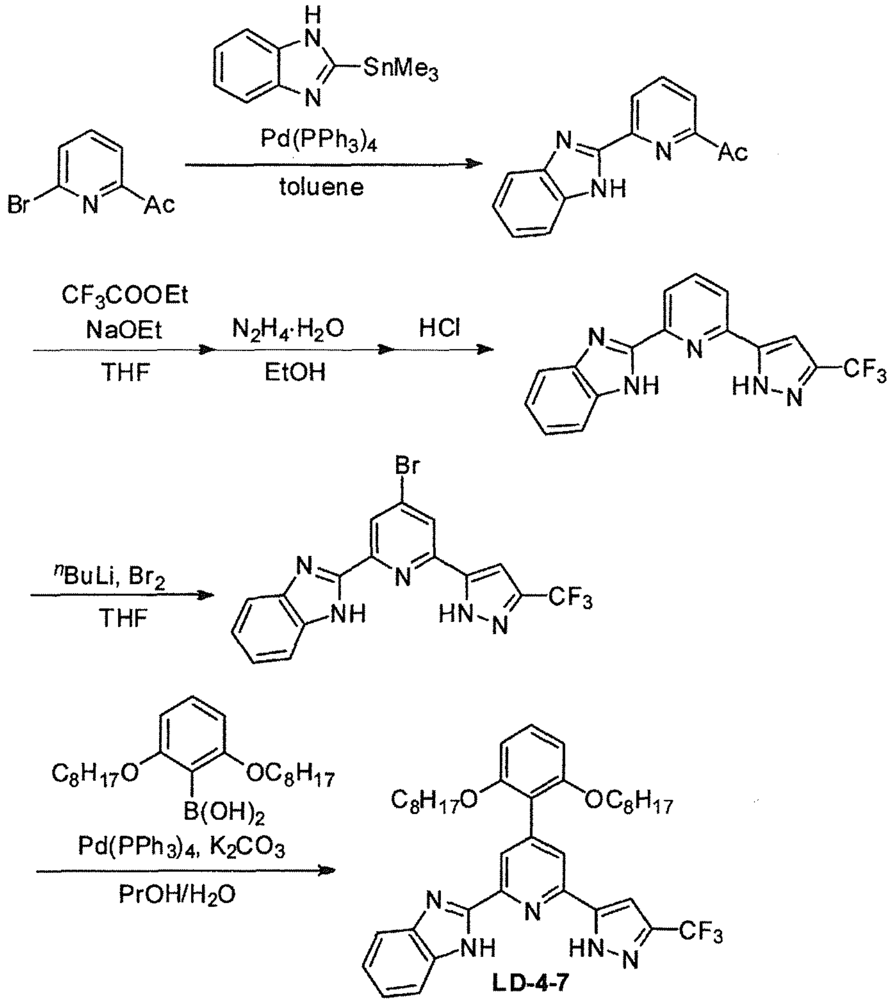


LD-4-7

Dyes 17 to 20 were synthesized using these ligands, and the ligand LD-1-1 synthesized in synthesis of the dye 1, the ligand LD-3-1 synthesized in synthesis of the dye 5 or the ligand LD-4-4 synthesized in synthesis of the dye 7 and by a method in accordance with the method for synthesizing the dye 1.

Each of these dyes (metal complex dye) was dissolved into ethanol, and λmax was measured using Spectrophotometer [U-4100 (trade name), manufactured by Hitachi High-Technologies Corporation]. These results are shown in Table 1 below.

Table 1

| Metal complex dye | | | | Absorption characteristics λmax |
|---|---|---|---|---|
| Dye No. | Ligand LD | Ligand LA | (Y)m Y | |
| Dye 1 | LD-1-1 | LA-1-1 | - | 692nm |
| Dye 2 | LD-1-25 | LA-1-1 | - | 687nm |
| Dye 3 | LD-2-5 | LA-1-1 | - | 674nm |
| Dye 4 | LD-2-8 | LA-1-1 | (C1)2 | 672nm |
| Dye 5 | LD-3-1 | LA-1-1 | - | 663nm |
| Dye 6 | LD-4-1 | LA-1-1 | - | 671nm |
| Dye 7 | LD-4-4 | LA-1-1 | - | 667nm |
| Dye 8 | LD-3-11 | LA-1-1 | C1 | 670nm |
| Dye 9 | LD-1-25 | LA-2-6 | - | 689nm |

(continued)

| Metal complex dye | | | | Absorption characteristics λmax |
|---|---|---|---|---|
| Dye No. | Ligand LD | Ligand LA | (Y)m Y | |
| Dye 10 | LD-2-8 | LA-2-1 | (Cl)2 | 687nm |
| Dye 11 | LD-3-1 | LA-2-13 | - | 674nm |
| Dye 12 | LD-4-4 | LA-2-13 | - | 677nm |
| Dye 13 | LD-1-1 | LA-3-6 | - | 690nm |
| Dye 14 | LD-2-5 | LA-3-8 | - | 688nm |
| Dye 15 | LD-4-4 | LA-3-8 | - | 687nm |
| Dye 16 | LD-3-1 | LA-3-11 | - | 693nm |
| Dye 17 | LD-1-1 | LA-5-1 | - | 686nm |
| Dye 18 | LD-4-7 | LA-5-1 | - | 692nm |
| Dye 19 | LD-4-4 | LA-5-3 | - | 689nm |
| Dye 20 | LD-3-1 | LA-5-4 | - | 695nm |

Example 1

Various kinds of pastes for forming photoconductor layer (a semiconductor layer) or a light-scattering layer of a semiconductor electrode that constitutes a photoelectrode were prepared, and dye-sensitized solar cells were produced using the pastes.

[Preparation of paste]

(Paste A)

Spherical $TiO_2$ particles (anatase, a mean particle diameter; 25 nm, hereinafter, referred to as spherical $TiO_2$ particles A) were put into a nitric acid solution, and the resultant mixture was stirred to prepare titania slurry. Next, a cellulose-based binder was added to the titania slurry as a thickening agent, and the resultant mixture was kneaded to prepare a paste.

(Paste 1)

Spherical $TiO_2$ particles A and spherical $TiO_2$ particles (anatase, a mean particle diameter; 200 nm, hereinafter, referred to as spherical $TiO_2$ particles B) were put into a nitric acid solution, and the resultant mixture was stirred to prepare titania slurry. Next, a cellulose-based binder was added to the titania slurry as a thickening agent, and the resultant mixture was kneaded to prepare a paste (a mass of $TiO_2$ particles A : a mass of $TiO_2$ particles B = 30 : 70).

(Paste 2)

Rod-shaped $TiO_2$ particles (anatase, diameter; 100 nm, aspect ratio; 5, hereinafter, referred to as rod-shaped $TiO_2$ particles C) were mixed with the paste A to prepare a paste having a mass of rod-shaped $TiO_2$ particles C: a mass of the paste A = 30 : 70.

According to the procedure described below, a photoelectrode having the same configuration as that of the photoelectrode 12 shown in Fig. 5 of JP-A-2002-289274 was produced, and using the photoelectrode, a dye-sensitized solar cell of a scale of 10 mm × 10 mm having the same configuration as that of the dye-sensitized solar cell 20 shown in Fig. 3 of JP-A-2002-289274 except for the photoelectrode, was produced. The specific configuration thereof was shown in Fig. 2 attached to the present application. In Fig. 2 of the present application, 20 represents a dye-sensitized solar cell, 41 represents a transparent electrode, 42 represents a semiconductor electrode, 43 represents a transparent electrically-conductive film, 44 represents a substrate, 45 represents a semiconductor layer, 46 represents a light-scattering layer, 40 represents a photoelectrode, CE represents a counter electrode, E represents an electrolyte, and S represents a spacer.

A transparent electrode in which a fluorine-doped $SnO_2$ electrically-conductive film (thickness: 500 nm) was formed on a glass substrate, was provided. On this $SnO_2$ electrically-conductive film, the paste 1 was applied to by screen printing, followed by drying. Then, the paste was calcined under the conditions of 450°C in the air. Further, by repeating this screen printing and calcination using the paste 2, the semiconductor electrode having the same configuration as that of the semiconductor electrode 42 shown in Fig. 2 of the present application (the area of the light-receiving face 10 mm $\times$ 10 mm; the layer thickness 15 $\mu$m; the layer thickness of the semiconductor layer 10 $\mu$m; the layer thickness of the light-scattering layer 5 $\mu$m; and the content of the rod-shaped $TiO_2$ particles C contained in the light-scattering layer 30% by mass) were formed on the $SnO_2$ electrically-conductive film. Thus, the photoelectrode, which did not contain the dye, was prepared.

Next, a dye was adsorbed onto a semiconductor electrode (precursor of a dye-adsorbed electrode) as follows. First, absolute ethanol dehydrated over magnesium ethoxide was used as a solvent to dissolve thereinto a metal complex dye described in Table 2 below to be 3 $\times$ $10^{-4}$ mol/L, and further, as a co-adsorbent, 50 mol of equimolar mixture of chenodeoxycholic acid and cholic acid was added to 1 mol of metal complex dye to prepare each dye solution. Next, the semiconductor electrode was immersed into this solution to complete a photoelectrode 40 in which about 1.5 $\times$ $10^{-7}$ mol/$cm^2$ of dye was adsorbed onto the semiconductor electrode, respectively.

Then, a platinum electrode (thickness of Pt thin film, 100nm) having the same shape and size as those of the photoelectrode as a counter electrode, and an iodine-based redox solution containing: 0.05M of iodine, 0.01M of lithium iodide, 0.6M of 1-propyl-3-methylimidazolium iodide and 4-tert-butylpyridine, as an electrolyte E, were prepared. Further, a spacer-S (trade name: "Surlyn") manufactured by DuPont, which had a shape matching to the size of the semiconductor electrode, was provided. As shown in Fig. 3 of JP-A-2002-289274, the photoelectrode 40 and the counter electrode CE were arranged to face each other, with the spacer-S interposed therebetween, followed by filling the electrolyte in the inside thereof. Thus, a dye-sensitized solar cell (cell A) was completed. The performance evaluation of the solar cell was conducted.

<Test method>

Performance of cell characteristics were determined, as described below, to investigate durability, a rate of dye adsorption to and desorption from titanium dioxide, solution stability of a metal complex dye and a change of absorption characteristics of the dye depending on a dye concentration.

Cell characteristic test

Photoelectric conversion efficiency $\eta$ (%), together with short-circuit current density Jsc (mA/$cm^2$), open-circuit voltage Voc (mV), and fill factor FF of each of the dye-sensitized solar cells (cell A), were measured, with irradiating a pseudo sunlight of 1,000 W/$m^2$ from a xenon lamp through an AM 1.5 filter, using a solar simulator (WXS-85H, manufactured by WACOM). The current-voltage characteristics were measured, using an I-V tester.

- Photoelectric conversion efficiency $\eta$ (%) -

The photoelectric conversion efficiency $\eta$ (%) obtained as described above was evaluated based on the following criteria.

(Evaluation criteria)

AA: 9.0% or more but 10.0% or less
A: 8.0% or more but less than 9.0%
B: less than 8.0%

- Short-circuit current density Jsc (mA/$cm^2$) -

The short-circuit current density Jsc (mA/$cm^2$) obtained as described above was evaluated based on the following criteria.

(Evaluation criteria)

AA: 20mA/$cm^2$ or more but 21mA/$cm^2$ or less
A: 19mA/$cm^2$ or more but less than 20mA/$cm^2$
B: less than 19mA/$cm^2$

- Open-circuit voltage Voc (V) -

The open-circuit voltage Voc (V) obtained as described above was evaluated based on the following criteria.

(Evaluation criteria)

AA: 0.65V or more but 0.68V or less
A: 0.60V or more but less than 0.65V
B: less than 0.60V

- Standard deviation of open-circuit voltage Voc (V) -

In order to investigate a deviation of performance between preparation lots of the dye-sensitized solar cell, the dye-sensitized solar cells (cell A) were repeatedly prepared 10 times to each metal complex dye in the same manner as above. An open-circuit voltage Voc (V) of each of the thus-prepared cells was measured, and a standard deviation of the open-circuit voltage Voc (V) was calculated.
A standard deviation value thereof was evaluated based on the following criteria.

(Evaluation criteria)

A: 0.010 or less
B: more than 0.010

- Durability -

The durability was evaluated in terms of rate of decrease in open-circuit voltage Voc (V) in heat resistance of the dye-sensitized solar cell.
The open-circuit voltage Voc (V) of the dye-sensitized solar cell (cell A) prepared as above was measured as above. Then, regarding durability, the open-circuit voltage Voc (V) with the lapse of time at 80°C for 300 hours in the dark was measured, to obtain a rate of decrease (%). The rate of decrease (%) was calculated according to the following expression. The rate of decrease (%) was evaluated based on the following criteria, according to [(Initial open-circuit voltage - Open-circuit voltage with the lapse of time in the dark)/Initial open-circuit voltage] x 100.

(Evaluation criteria)

AA: 0.0% or more but 2.5% or less
A: 2.5% or more but less than 6.0%
B: 6.0% or more

- Desorption rate -

For evaluation of adsorption power of a metal complex dye onto the titanium dioxide surface, a desorption rate of the metal complex dye from the titanium dioxide surface was used as an index.
The desorption rate of the metal complex dye was calculated by means of a Quartz Crystal microbalance with Dissipation monitoring (QCM-D) intermolecular interaction measuring apparatus E1 (manufactured by Meiwafosis).
Paste A (anatase, average particle size: 25 nm) was printed by screen printing (film thickness: 20 $\mu$m) on a gold sensor (manufactured by Meiwafosis) for use for the QCM-D. By calcining the thus-printed gold sensor at 450°C for 1 hour in the air, to prepare a gold sensor having a semiconductor layer adsorbed thereon.

The thus-prepared sensor was installed in the QCM-D intermolecular interaction measuring apparatus, and 0.2 mM of a dye solution (DMF/t-BuOH=1/1) was flowed therein, to make the dye adsorb on the semiconductor layer in a dye adsorption amount of a predetermined value (200 μg/cm$^2$). The dye adsorption amount was calculated from a resonance frequency shift (ΔF) of a quartz oscillator according to the following Sauerbrey equation.

$$\Delta F = -2 \times F_0^2 \times \Delta m / A(\mu \times P)^{1/2}$$

In the formula, $F_0$ represents a single frequency of a quartz oscillator, Δm represents a mass change, A represents a piezoelectric active area of the Au electrode, and μ and P represents quartz density and modulus of rigidity, respectively.

Then, by flowing the dye solution into the electrolyte E at 80°C for 4 hours, to measure desorption amount of the dye. Desorption amount of the dye was also calculated according to the Sauerbrey equation. The desorption rate was calculated from the resulting desorption amount of the dye. The rate obtained was evaluated based on the following criteria.

(Evaluation criteria)

AA: 0.0 μg/cm$^2$/hr or more but less than 2.5 μg/cm$^2$/hr
A: 2.5 μg/cm$^2$/hr or more but less than 10.0 μg/cm$^2$/hr
B: 10.0 μg/cm$^2$/hr or more

- Adsorption rate -

The adsorption rate of the metal complex dye onto a titanium dioxide surface was measured as follows.

The gold sensor having a semiconductor layer adsorbed thereon, which was prepared for the measurement of desorption rate, was installed in the QCM-D intermolecular interaction measuring apparatus, and by flowing therein 0.2 mM of a dye solution (DMF/t-BuOH=1/1), to make the dye adsorb on the semiconductor layer in a given amount (0.20 mg/cm$^2$). The dye adsorption amount was calculated according to the Sauerbrey equation. The adsorption rate of the dye was determined from the time period until the dye adsorption amount became a predetermined value. The rate obtained was evaluated based on the following criteria.

(Evaluation criteria)

AA: 100 μg/cm$^2$/hr or more but 250 μg/cm$^2$/hr or less
A: 50 μg/cm$^2$/hr or more but less than 100 μg/cm$^2$/hr
B: 10 μg/cm$^2$/hr or more but less than 50 μg/cm$^2$/hr
C: 0 μg/cm$^2$/hr or more but less than 10 μg/cm$^2$/hr

- Solution stability -

Each of dye solutions was prepared, in which the respective metal complex dye shown in Table 2 was adjusted to 34 μM, with, as a solvent, anhydrous ethanol dehydrated over magnesium ethoxide. Each of the dye solutions was encapsulated in a sealable cell, and light of 70,000 Lx was irradiated thereto using a merry-go-round-type light-resistance tester (III(N)-500W, manufactured by Eagle Engineering), to observe attenuation of λmax with the lapse of time, to evaluate dye stability as follows.

A 0.1 mM solution before light irradiation was diluted with a given quantity of a solvent so that absorbance (Abs.) of λmax would become 1. This was defined as dye residual ratio of 100%. Separately, a sample after irradiation for 480 hours was diluted by adding thereto the same quantity of the solvent, and a value multiplying the absorbance (Abs.) of λmax of the thus-diluted sample after irradiation for 480 hours by 100 was defined as a dye residual ratio at the time. Measurement was carried out using a spectrophotometer (U-4100 (trade name), manufactured by Hitachi High-Technologies). The results obtained were evaluated based on the following criteria.

(Evaluation criteria)

AA: 95% or more but 100% or less
A: 90% or more but less than 95%
B: 80% or more but less than 90%
C: 0% or more but less than 80%

- λmax Shift -

The titanium oxide paste 2 was printed on a FTO substrate by a screen printing, to a film thickness of 5μm as a semiconductor layer, and the thus-printed matter was immersed in 1.0 mM of the respective dye solution for a period of time from 30 minutes to 12 hours, to adsorb the dye thereon, to quantitate a dye adsorption amount. The dye adsorption amount was quantitated, by desorbing a dye with a 1 normal tetrabutylammonium hydroxide methanol solution, on a high-performance liquid chromatography. Transmission spectra of separately prepared cells under the conditions such that a dye adsorption density would become 70 $\mu g/cm^2$ or 140 $\mu g/cm^2$, were measured with respect to each dye, to observe a change of spectrum shape and a wavelength shift of λmax. Measurement was carried out using a spectrophotometer (U-4100, manufactured by Hitachi High-Technologies Corporation).

(Measuring conditions for high-performance liquid chromatography (HPLC))

Equipment used: System controller SCL-10AVP
Column oven: CTO-10ASVP
Detector: SPD-10AVVP
Degasser: DGU-14AM
Solution-sending unit: LC-10ADVP (trade name, manufactured by Shimadzu)
Column: YMC-Pack ODS-AM, model number AM-312
Size: 150 × 6.0 mm I.D. (manufactured by YMC Co., Ltd. Japan)
Flow rate: 0.75 mL/min
Eluent: THF/Water = 63/37 (containing 0.1% trifluoroacetic acid)
Temperature: 40°C
Detection wavelength: 254 nm

Then, λmax2 / λmax1, as a ratio of λmax (λmax2) when an amount of the dye was 140 $\mu g/cm^2$ to λmax (λmax1) when an amount of the dye was 70 $\mu g/cm^2$, was determined and evaluated based on the following criteria.

(Evaluation criteria)

AA: 0.99 or more but 1.00 or less
A: 0.95 or more but less than 0.99
B: 0.90 or more but less than 0.95
C: less than 0.90

These results are collectively shown in Table 2 below.

Table 2

| Sample No. | Metal complex dye | Jsc mA/cm$^2$ | Voc V | Voc Standard deviation | η % | Durability % | Desorption rate μg/cm$^2$/hr | Adsorption rate μg/cm$^2$/hr | Solution stability % | λmax shift λmax2/λmax1 | Remarks |
|---|---|---|---|---|---|---|---|---|---|---|---|
| 101 | Dye 1 | A | AA | A | AA | A | AA | A | B | B | This invention |
| 102 | Dye 2 | A | AA | A | AA | AA | AA | B | A | A | This invention |
| 103 | Dye 3 | A | A | A | A | A | AA | B | B | B | This invention |
| 104 | Dye 4 | A | A | A | A | A | AA | B | B | B | This invention |
| 105 | Dye 5 | A | AA | A | A | A | AA | A | B | B | This invention |
| 106 | Dye 6 | A | AA | A | AA | A | AA | B | A | B | This invention |
| 107 | Dye 7 | A | AA | A | AA | AA | AA | B | A | A | This invention |
| 108 | Dye 8 | A | A | A | A | A | AA | A | B | B | This invention |
| 109 | Dye 9 | A | AA | A | AA | AA | A | B | AA | A | This invention |
| 110 | Dye 10 | A | AA | A | A | A | A | B | AA | B | This invention |
| 111 | Dye 11 | A | A | A | A | A | A | B | AA | B | This invention |
| 112 | Dye 12 | A | AA | A | AA | AA | A | B | AA | A | This invention |
| 113 | Dye 13 | A | AA | A | AA | A | A | AA | AA | B | This invention |
| 114 | Dye 14 | A | A | A | A | A | A | AA | B | B | This invention |

(continued)

| Sample No. | Metal complex dye | Jsc mA/$cm^2$ | Voc V | Voc Standard deviation | η % | Durability % | Desorption rate μg/$cm^2$/hr | Adsorption rate μg/$cm^2$/hr | Solution stability % | λmax shift $\lambda max^2/\lambda max1$ | Remarks |
|---|---|---|---|---|---|---|---|---|---|---|---|
| 115 | Dye 15 | A | AA | A | AA | AA | A | AA | A | A | This invention |
| 116 | Dye 16 | A | A | A | A | A | A | AA | A | B | This invention |
| 117 | Dye 17 | AA | AA | A | AA | A | A | B | B | AA | This invention |
| 118 | Dye 18 | AA | A | A | A | AA | A | B | A | AA | This invention |
| 119 | Dye 19 | AA | AA | A | AA | AA | A | A | A | AA | This invention |
| 120 | Dye 20 | AA | A | A | A | A | A | B | A | AA | This invention |
| c01 | Comparative Dye 1 | B | B | B | B | B | B | C | C | C | Comparative Example |
| c02 | Comparative Dye 2 | B | B | B | B | B | B | C | C | C | Comparative Example |
| c03 | Comparative Dye 3 | B | B | B | B | B | B | C | C | C | Comparative Example |

Comparative dye 1

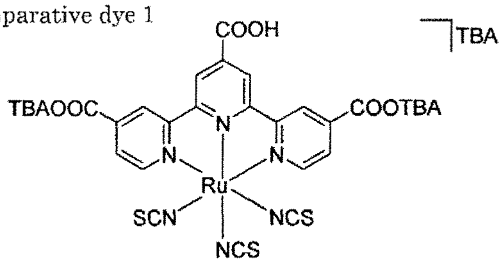

Comparative dye 2

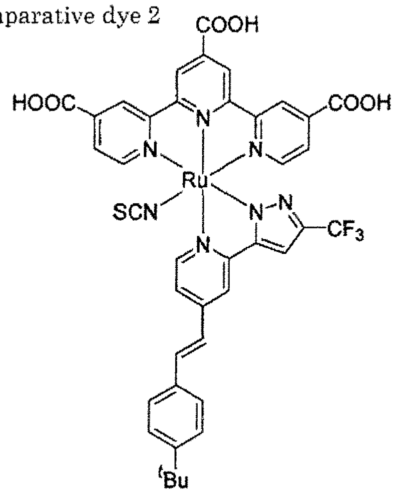

Comparative dye 3

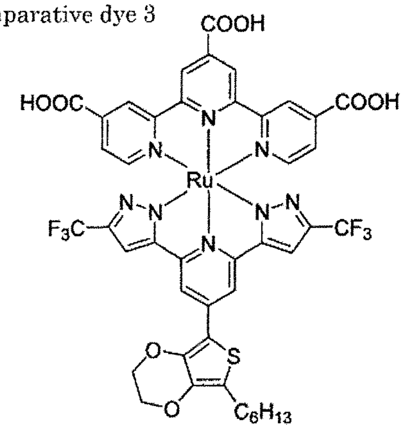

Here, the comparative dye 1 is N747, the comparative dye 2 is PRT4, and the comparative dye 3 is TF-3. TBA represents tetrabutylammonium and $^tBu$ represents a t -butyl group, respectively.

As is clear from the above-described results, in comparison with the conventional metal complex dye, all of the metal complex dyes according to the present invention are found to be ranked A or higher in the short-circuit current density Jsc, the open-circuit voltage Voc and the photoelectric conversion efficiency η to exhibit excellent cell characteristics, and simultaneously found to be low in the lot-to-lot difference in the cell characteristics (open-circuit voltage Voc). Furthermore, all of the metal complex dyes according to the present invention are found to be also excellent in the adsorption power and the durability against heat, and found to have a small change in dye-absorption characteristics even when an adsorption concentration is high.

In addition, the metal complex dye according to the present invention has a small change in λmax on the oxide semiconductor even when the dye adsorption amount increases, and considered to be difficult to form dye association between the dyes to be inefficient in light absorption. Moreover, one having a small shift in λmax tends to be excellent in the short-circuit current density Jsc, and when the association between the dyes is formed, electron injection efficiency into the oxide semiconductor is presumed to decrease.

Example 2

According to the following method, the dye-sensitized solar cell shown in Fig. 3 was prepared, by subjecting a photoelectrode to a CdSe quantum dot-making treatment, and employing an electrolyte with a cobalt complex.

An ethanol solution of titanium (IV) bis(acetylacetonato) diisopropoxide was sprayed 16 times onto the surface of FTO glass (manufactured by Nippon Sheet Glass, surface resistance: 8 $\Omega sq^{-1}$), followed by calcining at 450°C for 30 minutes or more. On this substrate, a transparent layer of about 2.1 μm composed of 20nm-$TiO_2$ and a light-scattering layer of about 6.2 μm composed of 60nm-$TiO_2$ (manufactured by Showa Titanium) were laminated by a screen printing, followed by subjecting to a posttreatment with a $TiCl_4$ aqueous solution, to prepare a FTO/$TiO_2$ film.

This FTO/$TiO_2$ film was soaked in a 0.03-M $Cd(NO_3)_2$ ethanol solution for 30 seconds in a glove bag under an inert gas atmosphere, followed by successively soaking in a 0.03-M selenide ethanol solution for 30 seconds. After that, the film was washed in ethanol for 1 minute or more, to remove an excessive precursor, followed by drying. These steps of: soaking → washing → drying, were repeated 5 times, to make the CdSe quantum dots 23 grow in semiconductor fine particles 22 of the titanium oxide layer. Then, the resultant film was subjected to a surface stabilization treatment with CdTe. In this manner, a CdSe-processed photoelectrode was prepared.

The selenide ($Se^{2-}$) was prepared in the system, by adding 0.068 g of $NaBH_4$ (so as to be 0.060 M concentration) to a 0.030 M $SeO_2$ ethanol solution, under Ar or $N_2$ atmosphere.

The CdSe-processed photoelectrode was soaked for 4 hours in the dye solution containing the metal complex

dye as shown in Table 1 (ex. 1=0.3 mM Z907 Na acetonitrile/t-butanol (1:1) solution; and ex. 2=0.1 mM SQ1 ethanol solution), to adsorb the metal complex dye as shown in Table 1 onto the photoelectrode. After that, the photoelectrode and the counter electrode 4 (a product obtained by chemically depositing Pt from a 2-propanol solution of hexachloroplatinate (0.05 M) on a FTO glass, at 400 °C for 20 minutes) were put together by sandwiching a 25 μm-thick Surlyn (manufactured by DuPont) ring between them, followed by sealing by thermal melting. An electrolyte using a cobalt complex (an acetonitrile/ethylene carbonate (4:6/v:v) solution of 0.75M $Co(o\text{-}phen)_3^{2+}$, 0.075M $Co(o\text{-}phen)_3^{3+}$, and 0.20M $LiClO_4$) was injected into the interspace 3 between the electrodes through an opening made in advance in the side of the counter electrode. After that, the opening was closed by heat with a BYNEL (manufactured by DuPont) sheet and a thin glass slide, to prepare a dye-sensitized solar cell (cell A').

Similarly, a dye-sensitized solar cell (cell B') was prepared, using the same iodine-based redox solution containing iodine and lithium iodide as in Example 1.

The cobalt complex added to the electrolyte was prepared according to a method described in Chemical Communications, Vol. 46, pages 8788 to 8790 (2010).

The performance of each of the thus prepared dye-sensitized solar cells was evaluated in a manner similar to the evaluation in Example 1, and thus all of the metal complex dyes according to the present invention were confirmed to be ranked A or higher in the short-circuit current density Jsc, the open-circuit voltage Voc and the photoelectric conversion efficiency η to exhibit excellent cell characteristics, and simultaneously confirmed to be low in the lot-to-lot difference in the cell characteristics (open-circuit voltage Voc).

Having described our invention as related to the present embodiments, it is our intention that the invention not be limited by any of the details of the description, unless otherwise specified, but rather be construed broadly within its spirit and scope as set out in the accompanying claims.

This application claims priority on Patent Application No. 2012-061232 filed in Japan on March 16,2012 which is entirely herein incorporated by reference.

REFERENCE SIGNS LIST

1 Electrically conductive support
2 Photoconductor layer

- 21 Metal complex dye (Dye)
- 22 Semiconductor fine particles
- 23 CdSe quantum dots
- 24 Co-adsorbent

3 Electrolyte layer
4 Counter electrode
5 Light-receiving electrode
6 Circuit
10 Photoelectric conversion element
100 System utilizing a dye-sensitized solar cell
M Electric motor (Electric fan)
20 Dye-sensitized solar cell
40 Photoelectrode
41 Transparent electrode
42 Semiconductor electrode
43 Transparent conductive film
44 Substrate
45 Semiconductor layer
46 Light-scattering layer
CE Counter electrode
E Electrolyte
S Spacer

## Claims

1. A metal complex dye represented by the following formula (I):

$M(LD)(LA)\cdot(Y)_{mY}$ formula (I)

wherein, in formula (I), M represents $Fe^{2+}$, $Ru^{2+}$ or $Os^{2+}$;
LD represents a tridentate ligand represented by the following formula (A);
LA represents a tridentate ligand represented by the following formula (B);
Y represents a counter ion required for neutralizing an electric charge; and
mY represents an integer of 0 to 2,

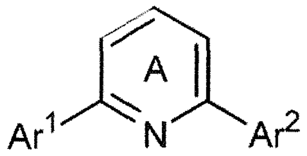
Formula (A)

wherein, in formula (A), $Ar^1$ and $Ar^2$ each independently represent a group selected from a benzene ring group, a pyrrole ring group, a pyrazole ring group, an imidazole ring group, a pyrazine ring group, a pyrimidine ring group, a pyridazine ring group, a triazole ring group, a triazine ring group, a tetrazole ring group, a quinoline ring group, an isoquinoline ring group, a quinazoline ring group, a thiazole ring group, an isothiazole ring group, an oxazole ring group, an isoxazol ring group, a furan ring group, a thiophene ring group, a pyrrolidine ring group, a piperidine ring group, a morpholine ring group, a piperazine ring group, a tetrahydrofuran ring group, a tetrahydropyran ring group, a 4H-pyran ring group, a 1,4-dihydropyridine ring group, a tetradehydromorpholine ring group, a tetradehydrothiomorpholine ring group and a group in which a benzene ring is condensed to the cyclic groups; provided that, when one of $Ar^1$ and $Ar^2$ is a pyrazole ring group, the other is selected from the above-described cyclic groups other than the pyrazole ring group; the cyclic group of $Ar^1$ and $Ar^2$ may be an anion in which a hydrogen atom is deprotonated; all of $Ar^1$, $Ar^2$ and a pyridine ring A may have a substituent other than a carboxyl group, and in a case where two or more substituents exist, these may be bonded to form a ring; $Ar^1$ and $Ar^2$ coordinate with M via a nitrogen atom having a lone electron pair, a nitrogen atom having an anion or a carbon atom having an anion, and

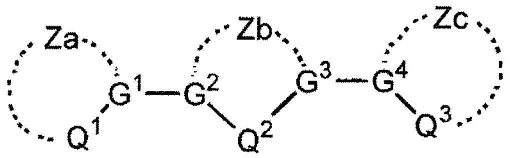
Formula (B)

wherein, in formula (B), Za, Zb and Zc each independently represent a group of atoms for forming a 5- or 6-membered ring; in which at least one of the rings formed by Za, Zb and Zc has an acidic group;
$Q^1$ to $Q^3$ each independently represent a nitrogen atom having a lone electron pair, a nitrogen atom having an anion, or a carbon atom having an anion;
$G^1$ to $G^4$ each independently represent a carbon atom or a nitrogen atom; and
each bond of $G^1$-$Q^1$, $G^2$-$Q^2$, $G^3$-$Q^2$ and $G^4$-$Q^3$ represents a single bond or a double bond.

2. The metal complex dye according to Claim 1, wherein LD is represented by the following formula (A1):

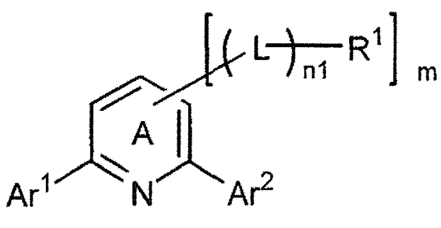
Formula (A1)

wherein, in formula (A1), $Ar^1$ and $Ar^2$ have the same meanings as those in formula (A);

L represents a divalent linking group; n1 represents an integer of 0 to 3;
$R^1$ represents an alkyl group, a cycloalkyl group, an alkenyl group, a cycloalkenyl group, an alkynyl group, an aryl group, a heterocyclic group, an alkoxy group, a cycloalkyloxy group, a cycloalkenyloxy group, an alkynyloxy group, an aryloxy group, a heterocyclic oxy group, an alkylthio group, a cycloalkylthio group, a cycloalkenylthio group, an alkynylthio group, an arylthio group, a heterocyclic thio group, an amino group, an alkylamino group, a cycloalkylamino group, an alkenylamino group, a cycloalkenylamino group, an arylamino group, a heterocyclic amino group, a silyl group, a silyloxy group or a halogen atom; and
m represents an integer of 0 to 3.

**3.** The metal complex dye according to Claim 2, wherein $R^1$ is represented by the following formula (A1-1) or (A1-2):

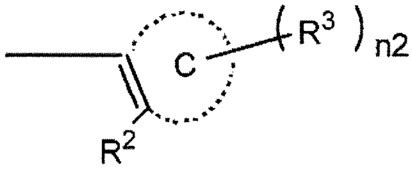

Formula (A1-1)

wherein, in formula (A1-1), ring C represents an aromatic ring or a heteroaromatic ring; $R^2$ and $R^3$ each independently represent a substituent; provided that the formula (A1-1) does not represent the following formula (A1-2);
n2 represents an integer of 0 to 4; when n2 is one or more, $R^2$ and $R^3$ may be bonded to form a ring; and when n2 is two or more, plural $R^3$'s may be bonded with each other to form a ring,

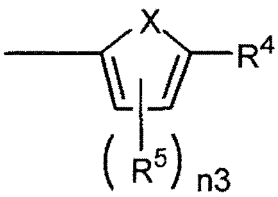

Formula (A1-2)

wherein, in formula (A1-2), X represents -O-, -N(Rz)-, -S- or -Se-;
$R^4$ represents a hydrogen atom, an alkyl group, an alkenyl group, an alkynyl group, an alkoxy group, an alkylthio group, an aryl group, an aryloxy group, an arylthio group, a silyloxy group, an amino group, a silyl group, a heterocyclic group or a halogen atom; Rz represents an alkyl group, an alkenyl group, an alkynyl group, an aryl group, a silyl group or a heterocyclic group;
$R^5$ represents an alkyl group, an alkenyl group, an alkynyl group, an alkoxy group, an alkylthio group, an aryl group, an aryloxy group, an arylthio group, a silyloxy group, an amino group, a silyl group, a heterocyclic group or a halogen atom;
n3 represents an integer of 0 to 2; when n3 is one or more, $R^4$ and $R^5$ may be bonded to form a ring; and when n3 is 2, and plural $R^5$'s may be bonded with each other to form a ring.

**4.** The metal complex dye according to any one of Claims 1 to 3,
wherein a coordinating atom of one of $Ar^1$ and $Ar^2$ is a carbon atom.

**5.** The metal complex dye according to any one of Claims 1 to 4,
wherein LA is represented by any one of the following formulas (B1) to (B8),

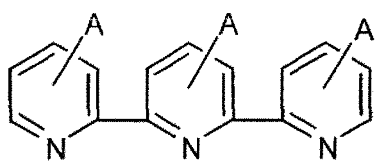

Formula (B1)

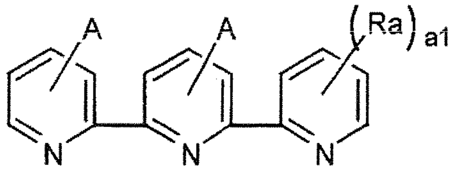

Formula (B2)

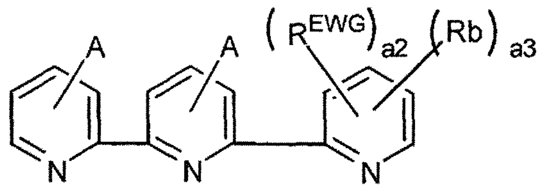

Formula (B3)

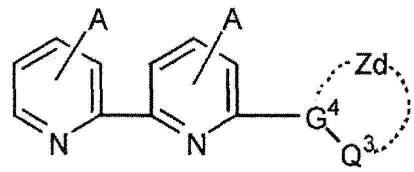

Formula (B4)

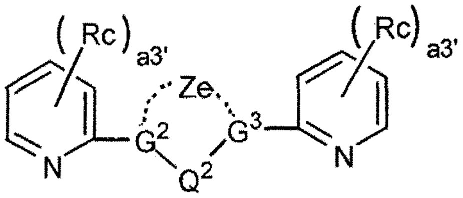

Formula (B5)

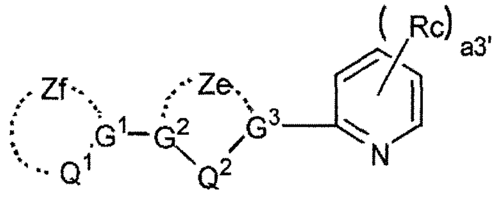

Formula (B6)

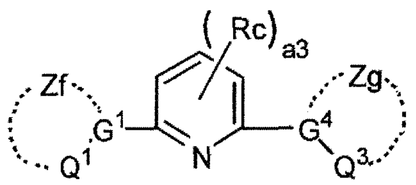

Formula (B7)

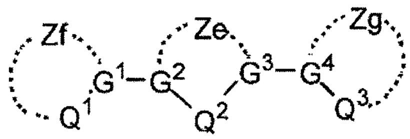

Formula (B8)

wherein $Q^1$ to $Q^3$ and $G^1$ to $G^4$ have the same meanings as those in formula (B), respectively;
Zd represent a group of atom for forming a benzene ring, a naphthalene ring, an anthracene ring, a phenanthrene ring, a pyrene ring, a perylene ring, a pyrrole ring, an indole ring, an imidazole ring, a benzoimidazole ring, a pyrazole ring, a pyrazine ring, a pyrimidine ring, a pyridazine ring, a triazole ring, a benzotriazole ring, a tetrazole ring, an indazole ring, a triazine ring, a purine ring, a quinoline ring, an isoquinoline ring, a quinazoline ring, a thiazole ring, a benzothiazole ring, an isothiazole ring, an oxazole ring, an isoxazole ring, a furan ring, a benzo[b]furan ring, a thiophene ring, a benzo[b]thiophene ring, a pyrrolidine ring, a piperidine ring, a morpholine ring, a piperazine ring, a tetrahydrofuran ring, a tetrahydropyran ring, a 4H-pyran ring, a 1,4-dihydropyridine ring, a tetradehydromorpholine ring, or a tetradehydrothiomorpholine ring;
Ze to Zg represent a group of atom for forming a 5- or 6-membered ring other than a pyridine ring;
A represents an acidic group;
Ra represents an aryl group, a heteroaryl group, an alkyl group, a cycloalkyl group, an alkoxy group, or a cycloalkoxy group; in which the alkyl group has a tertiary or quaternary carbon atom, and the alkoxy group has a tertiary or quaternary carbon atom, or a carbon atom directly binding to an oxygen atom of the alkoxy group is a secondary or tertiary carbon atom; a1 represents an integer of 1 or 2;
$R^{EWG}$ represents an electron-withdrawing group;
Rb and Rc represent a substituent;
a2 represents an integer of 1 to 4; a3 represents an integer of 0 to 3; a3' represents an integer of 0 to 4; in which the sum of a2 and a3 is an integer of 1 to 4; and
the ligands represented by any one of formulas (B5) to (B8) each have at least one acidic group.

**6.** The metal complex dye according to any one of Claims 1 to 5,
wherein Y in formula (I) is a halogen ion, an arylsulfonate ion, an aryldisulfonate ion, an alkylsulfate ion, a sulfate ion, a thiocyanate ion, a perchlorate ion, a tetrafluoroborate ion, a hexafluorophosphate ion, an acetate ion, a trifluoromethanesulfonate ion, an ammonium ion, an alkali metal ion or a hydrogen ion.

**7.** A photoelectric conversion element, comprising:

at least a photoconductor layer; and
an electrolyte,

wherein a semiconductor and the metal complex dye according to any one of Claims 1 to 6 are contained in the photoconductor layer.

**8.** The photoelectric conversion element according to Claim 7,
wherein a redox-based compound contained in the electrolyte is a cobalt complex.

**9.** A dye-sensitized solar cell, comprising the photoelectric conversion element according to Claim 7 or 8.

**10.** A dye adsorption liquid composition for a dye-sensitized solar cell, comprising 0.001 to 0.1 mass% of the metal complex dye according to any one of Claims 1 to 6 in an organic solvent, provided that the dye adsorption composition for a dye-sensitized solar cell contains 0.1 mass% or less of water.

**11.** A semiconductor electrode for a dye-sensitized solar cell, wherein a metal complex dye is carried on a surface of a semiconductor of a semiconductor electrode by using the liquid composition according to Claim 10.

**12.** A method of producing a dye-sensitized solar cell, comprising the step of:

making a surface of a semiconductor of a semiconductor electrode carry a metal complex dye by using the liquid composition according to Claim 10.

*Fig. 1*

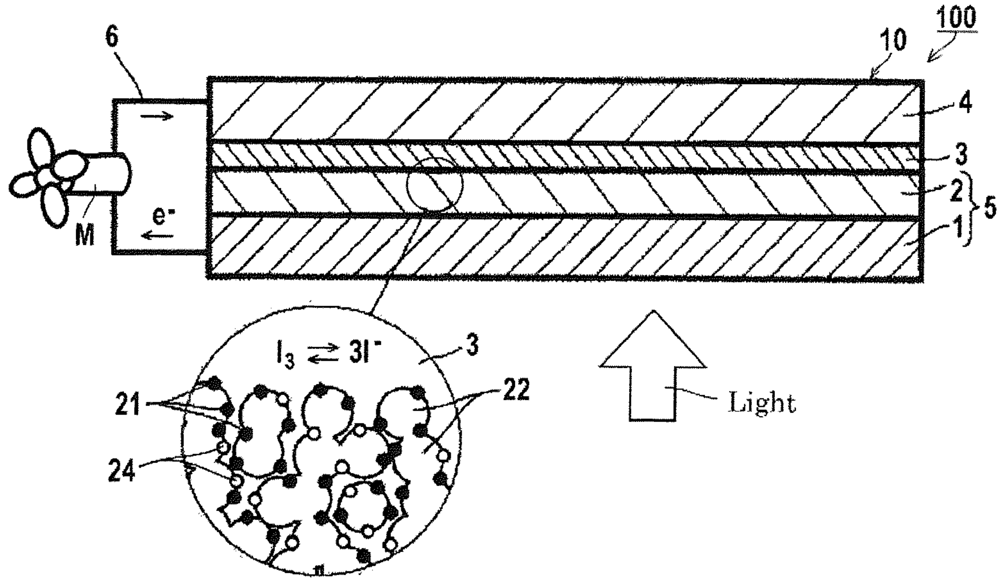


*Fig. 2*

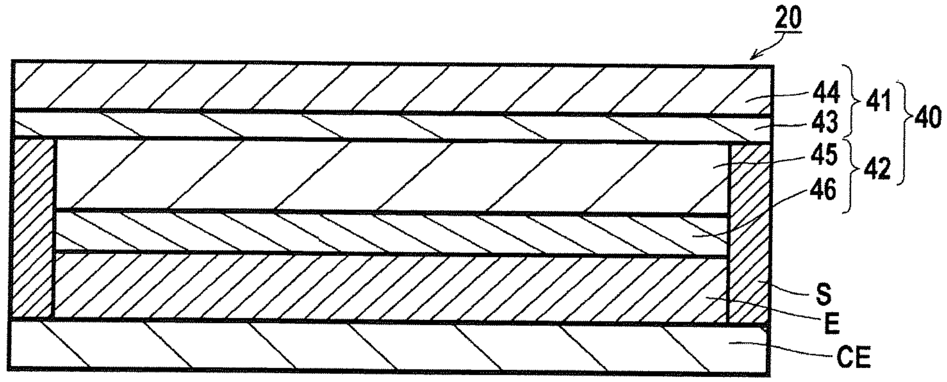


*Fig. 3*

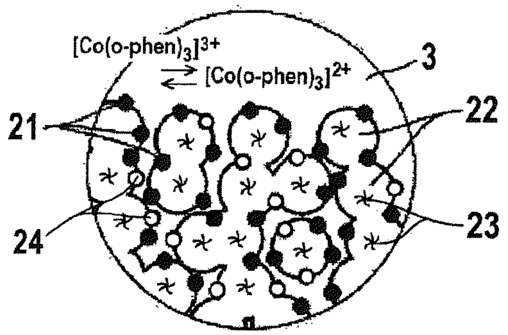

**INTERNATIONAL SEARCH REPORT**

International application No. PCT/JP2013/056724

A. CLASSIFICATION OF SUBJECT MATTER
*C09B57/10*(2006.01)i, *H01L31/04*(2006.01)i, *H01M14/00*(2006.01)i

According to International Patent Classification (IPC) or to both national classification and IPC

B. FIELDS SEARCHED

Minimum documentation searched (classification system followed by classification symbols)
C09B57/10, H01L31/04, H01M14/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | SCHULZE,B. et al., Ruthenium(II) Photosensitizers of Tridentate Click-Derived Cyclometalating Ligands: A Joint Experimental and Computational Study, Chemistry - A European Journal, 2012, Vol.18, No.13, p.4010-4025, Published online: February 29, 2012 | 1-3,5-7,9-12<br>8<br>4 |
| X<br>Y<br>A | JP 2009-67976 A (JSR Corp.), 02 April 2009 (02.04.2009), claims; paragraphs [0097] to [0110] & EP 2036955 A1 | 1-3,5-7,9-12<br>8<br>4 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 June, 2013 (05.06.13) | 18 June, 2013 (18.06.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2013/056724

C (Continuation). DOCUMENTS CONSIDERED TO BE RELEVANT

| Category* | Citation of document, with indication, where appropriate, of the relevant passages | Relevant to claim No. |
|---|---|---|
| X<br>A | TERADA,K. et al., Electric conduction properties of self-assembled monolayer films of Ru complexes with disulfide/phosphonate anchors in a Au-(molecular ensemble)-(Au nanoparticle) junction, Chemistry Letters, 2009, Vol.38, No.5, p.416-417, Supporting information | 1-3,5,6<br>4,7-12 |
| X<br>A | JP 2008-66542 A (Chuo University), 21 March 2008 (21.03.2008), paragraphs [0044] to [0051] (Family: none) | 1-3,5-7<br>4,8-12 |
| X<br>A | WANG,K. et al., Effect of subphase pH and metal ion on the molecular aggregates of amphiphilic Ru complexes containing 2,2':6',2''-terpyridine-4'-phosphonic acid at the air-water interface, Langmuir, 2002, Vol.18, No.9, p.3528-3536 | 1-3,5,6<br>4,7-12 |
| X<br>A | FUKUO,T. et al., Matrix-assisted laser desorption/ionization time-of-flight mass spectrometry of self-assembled monolayers of ruthenium complexes on gold, Rapid Communications in Mass Spectrometry, 2000, Vol.14, No.14, p.1301-1306 | 1-3,5,6<br>4,7-12 |
| Y<br>A | JP 2011-29131 A (Kyocera Corp.), 10 February 2011 (10.02.2011), paragraph [0039] (Family: none) | 8<br>1-7,9-12 |
| Y<br>A | JP 2010-277990 A (JSR Corp.), 09 December 2010 (09.12.2010), paragraph [0032] & CN 101877277 A | 8<br>1-7,9-12 |
| Y<br>A | JP 2011-195745 A (National Institute of Advanced Industrial Science and Technology), 06 October 2011 (06.10.2011), paragraph [0023] (Family: none) | 8<br>1-7,9-12 |
| P,X | US 2012/0073660 A1 (CHI Yun), 29 March 2012 (29.03.2012), claims; paragraphs [0049] to [0059] & TW 201213309 A | 1-12 |
| P,X<br>P,A | BROWN,D.G. et al., Stabilization of Ruthenium Sensitizers to TiO2 Surfaces through Cooperative Anchoring Groups, Journal of the American Chemical Society, 2013, Vol.135, No.5, p.1692-1695 | 1-3,5-12<br>4 |

Form PCT/ISA/210 (continuation of second sheet) (July 2009)

**INTERNATIONAL SEARCH REPORT**

International application No.
PCT/JP2013/056724

C (Continuation). DOCUMENTS CONSIDERED TO BE RELEVANT

| Category* | Citation of document, with indication, where appropriate, of the relevant passages | Relevant to claim No. |
|---|---|---|
| P,X<br>P,A | WU,K.-L. et al., Engineering of Osmium(II)-Based Light Absorbers for Dye-Sensitized Solar Cells, Angewandte Chemie, International Edition, 2012, Vol.51, No.23, p.5642-5646 | 1-3,5-12<br>4 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100258175 A1
- JP 4298799 B
- JP 2001291534 A
- US 4927721 A
- US 4684537 A
- US 5084365 A
- US 5350644 A
- US 5463057 A
- US 5525440 A
- JP H7249790 A
- JP 2004220974 A
- JP 2008135197 A
- WO 9518456 A
- JP 8259543 A
- JP 7249790 A
- JP 2005135902 A
- JP 2001160425 A
- JP 2003123859 A
- JP 2002260746 A
- JP 4260494 B
- JP 2004146425 A
- JP 2000340269 A
- JP 2002289274 A
- JP 2004152613 A
- JP 9027352 A
- JP 2000090989 A
- JP 2003217688 A
- JP 2002367686 A
- JP 2003323818 A
- JP 2001043907 A
- JP 2005085500 A
- JP 2004273272 A
- JP 2000323190 A
- JP 2000228234 A
- JP 2001266963 A
- JP 2001185244 A
- JP 2001525108 T
- JP 2001203377 A
- JP 2000100483 A
- JP 2001210390 A
- JP 2002280587 A
- JP 2001273937 A
- JP 2000285977 A
- JP 2001320068 A
- JP 2012061232 A


### Non-patent literature cited in the description


- *Angew. Chem. Int. Ed.,* 2011, vol. 50, 2054-2058
- *Chem. Commun.,* 2009, 5844-5846
- *Denki Kagaku (Electrochemistry),* 1997, vol. 65 (11), 923
- *Chemical Communications,* 2010, vol. 46, 8788-8790